# EUROPEAN PATENT APPLICATION

(11) **EP 4 527 828 A1**
(43) Date of publication of application: **26.03.2025**
(21) Application number: 23819089.6
(22) Date of filing: 06.06.2023
(51) Int. Cl.: C07C 49/755, C07C 15/14, A61K 31/12, A61K 31/165, A61P 35/00, A61P 3/00, A61P 25/16, A61P 29/00

(54) **BIPHENYL COMPOUND, PHARMACEUTICAL COMPOSITION, METHOD FOR PREPARING SAME, AND USE THEREOF**

(30) Priority: 09.06.2022 CN 202210650739
(71) Applicant: Longivitron (Suzhou) Biotechnology Co., Ltd, Suzhou, Jiangsu 215000 (CN)
(72) Inventor: LIU, Xiaoyu, Suzhou, Jiangsu 215000 (CN); CHEN, Xiaoguang, Suzhou, Jiangsu 215000 (CN); LI, Yan, Suzhou, Jiangsu 215000 (CN); ZHANG, Xiaoxi, Suzhou, Jiangsu 215000 (CN); ZHAO, Yanshi, Suzhou, Jiangsu 215000 (CN)
(74) Representative: Buzzi, Notaro & Antonielli d'Oulx S.p.A.
(86) International application number: PCT/CN2023/098475
(87) International publication number: WO 2023/236924

(57) **Abstract**

Provided are a biphenyl compound, a pharmaceutical composition, a method for preparing same, and use thereof. The biphenyl compound has a structure represented by formula I. The provided biphenyl compound can have relatively good binding to multiple target proteins, has relatively good bioavailability, and can be used for preparing drugs for preventing or treating tumors, autoimmune diseases, inflammatory diseases, and neurodegenerative diseases or anti-aging drugs.

## Description

### TECHNICAL FIELD

The present application belongs to the technical field of medicine and, in particular, relates to a biphenyl compound, a pharmaceutical composition, a method for preparing same, and use thereof.

### BACKGROUND

Medical data suggest that inflammation is one of the risk factors for tumors. For example, chronic hepatitis may be the direct cause of liver cancer, infection of the stomach with *Helicobacter pylori* tends to increase the risk of gastric cancer, and a relatively large number of patients suffer from cervical cancer due to papillomavirus infection. There are other factors. For example, autoimmune enteropathy is closely related to colon cancer, and PM2.5 particulate matter in the air is also a vicious factor behind lung cancer. A tumor is defined as a non-communicable disease like heart disease, chronic respiratory disease or diabetes. Most of the tumors are chronic diseases and progress relatively slowly. The correlation between inflammation and tumors was first proposed by Galenus 1800 years ago, and many studies have proven that persistent inflammation may cause lesions to progress from infections or autoimmune inflammation to tumors.

Biphenyl compounds are widely found in active natural products, have a variety of biological activities such as antiviral activity, antioxidant activity, anti-inflammatory activity, anti-tumor activity, anti-spasmodic activity, and anti-metabolic activity, and thus have broad research prospects. The applicants have been engaged in the study on the structure optimization of such natural products and the structure-activity relationship with the anti-tumor activity, treatment of autoimmune diseases, anti-inflammatory activity, and anti-metabolic activity. The structure modification of the natural products can help to discover precursors with stronger tumor inhibition activity, stronger therapeutic activity for autoimmune diseases and inflammatory diseases, and lower toxicity than the original natural products, and such precursors can be further used as anti-tumor, anti-inflammatory immune, and anti-metabolic drugs.

The potential anti-tumor and anti-inflammatory immune targets of the biphenyl compounds designed in the content of the present application are summarized below.

Epidermal growth factor receptor (EGFR): Mutational activation of EGFR is an important factor leading to the abnormal biological activities of tumor cells. The T790M mutation in EGFR is caused when a base pair changes from cytosine (C) to thymine (T), that is, the threonine at site 790 in the tyrosine kinase function of EGFR is replaced by methionine. Such a mutation can re-activate the EGFR, thereby leading to resistance to tyrosine kinase inhibitors

(TKIs). After such a mutated protein is docked with the aforementioned compounds and the derivatives thereof by computer-aided design, it has been found that all resulting compounds scored highly, suggesting that the biphenyl compounds can serve as candidates for the targets.

Vascular endothelial growth factor (VEGF): VEGF is a signal protein that stimulates the formation of blood vessels in cells and has the function of promoting vasculogenesis and angiogenesis. VEGFs bind to vascular endothelial growth factor receptors (VEGFR, also known as tyrosine kinase receptors) on the surface of the cell membrane and produce biological effects through a series of signaling pathways, ultimately leading to angiogenesis.

Histone-lysine N-methyltransferase enzyme EZH2: EZH2 is an enzyme encoded by the human EZH2 gene. It has been identified that two transcript variants transcribed from the aforementioned gene encode different isoforms; gene sequence alterations are fundamentally different from epigenetic modification abnormalities. Once the DNA sequence is mutated, the gene is difficult to repair or the mutated gene product is also difficult to eliminate. However, the epigenetic modification abnormalities can potentially be reversed by inhibitors of chromatin-modifying enzymes associated therewith. Therefore, it is important to clarify the mechanism of action of epigenetic modification enzymes in tumor cells to provide corresponding therapeutic means to prevent epigenetic modification mutations. In 2020, the FDA approved Epizyme's EZH2 inhibitor Tazemetostat for the treatment of metastatic or locally advanced epithelioid sarcoma. Currently, there are five drugs in Phase I/II clinical trials.

Histone deacetylase (HDAC): HDACs are a class of proteases and play an important role in the structural modification of chromosomes and the regulation of gene expression. In general, histone acetylation facilitates the dissociation of DNA and histone octamer. The structure of nucleosomes is relaxed, thereby enabling various transcription factors and co-transcription factors to specifically bind to DNA binding sites and activating gene transcription. In the nucleus, histone acetylation and histone deacetylation are in dynamic equilibrium and are jointly regulated by histone acetyltransferases (HATs) and histone deacetylases (HDACs).

Src kinase: Src kinases are a type of non-receptor protein kinases, are widely found in cancer cells, and play an important role in various processes of cell growth and proliferation, such as gene transcription, cell differentiation, migration, angiogenesis, prevention of apoptosis, etc. The study of Src inhibitors has become a hot spot in the research of anti-tumor drugs. Currently, a series of Src inhibitors are undergoing clinical trials.

Cancer immunotherapy is an increasingly effective therapeutic strategy. T cells play a key role in immunotherapy, and numerous immune checkpoints are treasures to be discovered. Since the efficacy of CTLA-4 and PD-1/PD-L1 monoclonal antibodies has been affirmed, there has been intense competition on the track of monoclonal antibodies. Therefore, more attention should be paid to other immune checkpoints and targets whose functions determine the efficacy of immunotherapy.

The survival and development of T cells are affected by T cell receptor (TCR) signals, and the TCR signaling pathway is dependent on the Src family kinase (SFK). Lck is an important member of SFK and is expressed throughout most of the life cycle of T cells. Moreover, Lck plays an important role in activating the TCR signaling pathway to activate T cells. Csk is a key regulator of SFK and inactivates Lck by phosphorylation on Lck (Tyr505), and Lck inhibits the activation of T cells via TCRs. Therefore, Csk and p-Lck (Tyr505) may be effective targets for future immunomodulatory treatment.

CD73 is a 5'-nucleotidase and can hydrolyze extracellular adenosine monophosphate (AMP) to adenosine. Adenosine, as a powerful immunosuppressive molecule, can inhibit the activation of CD8-positive T cells and thus help cancer cells to escape from the "hunting" of T cells. Tumor-infiltrating natural killer (NK) cells upregulate the expression of CD73, and the incidence of these CD73⁺ NK cells correlates with the tumor size of a breast cancer patient. It is supported by research that tumors can hijack NK cells for immune evasion and that CD73 expression defines an inducible NK cell population and has immunomodulatory properties in the tumor microenvironment.

KIR: Killer cell immunoglobulin-like receptors (KIRs) are expressed on the cell membrane of NK cells and a minority of T cells and can specifically recognize major histocompatibility (MHC) class I molecules on the cell surface and thus exert immunoregulation functions, thereby playing a role in the occurrence and development of tumors and immune-inflammatory diseases.

LAG-3: Lymphocyte activation gene 3 (LAG-3) is a negative immunoregulation molecule receptor that is distributed in activated T cells, NK cells, and dendritic cells and can bind to MHC-II molecules and FGL1. LAG-3 has the function of maintaining the stability of the internal environment and participating in immunoregulation and is closely related to the occurrence and development of tumors, immune-inflammatory diseases, and metabolic diseases. Currently, there have been several LAG3 monoclonal antibodies in clinical trials.

4-1BB, also known as CD137, is a member of TNF family and is expressed on the surface of activated T cells. 4-1BB is an inducible T-cell surface receptor. 4-1BB, as well as its ligands, is another important co-stimulatory molecule in addition to the CD28/B7 co-stimulatory signaling pathway.

PI3K-AKT-mTOR pathway: Phosphatidylinositol 3-kinase (PI3K) is a member of the lipid kinase family, participates in metabolic processes such as cellular glycolysis and lipogenesis, and can regulate tumor angiogenesis. The PI3K/Akt/mTOR signaling pathway induces tumorigenesis and can participate in autophagy through the following mechanisms, for example, by inhibition of autophagy by PI3K-specific inhibitor trimethyladenine (3-MA); can inhibit apoptosis through multiple mechanisms, for example, by inhibition of conformational changes in Bax and phosphorylation of components of other apoptotic structures such as Bad and caspases9 at the level of mitochondrial membrane potential; can down-regulate the expression of tumor suppressor protein p53 in the nucleus.

ACAT1 belongs to the specific thiolase superfamily and is known as acetoacetyl-CoA thiolase or acetyl-CoA acetyltransferase (ACAT). It has been reported that the expression of ACAT1 in tumor cells is usually aberrant, and ACAT1 plays a crucial role in the occurrence and development of tumors. The up-regulation mechanism of ACAT1 activity in different human cancer cells is worth studying. High expression of ACAT1 decreases the overall survival of the cancer cells. The reuse of the ketone bodies generated from the overexpression of ACAT1 in MDA-MB-231 human breast cancer cells drives the progression and metastasis of tumors. Therefore, the study of biphenyl compounds targeting ACAT1 may lead to the discovery of new anti-tumor drugs.

Although biphenyl compounds have been proven to have a wide range of activities as described above, their exact mechanism of action and structure-activity relationships still need to be further studied, and it is necessary to synthesize their structural derivatives and study their drug mode of action as well as their use in the treatment of diseases such as inflammation-related diseases, tumors, autoimmune diseases, neurodegenerative diseases, metabolic diseases, and aging.

### SUMMARY

The present application provides a biphenyl compound, a pharmaceutical composition, a method for preparing the same, and use thereof. The biphenyl compound and isomers, pharmaceutically acceptable salts, prodrugs, polycrystals or co-crystals thereof provided by the present application may be used in the preparation of a drug for preventing and/or treating a tumor, an autoimmune disease, an inflammatory disease, a neurodegenerative disease or a metabolic disease drug or an anti-aging drug.

In a first aspect, the present application provides a biphenyl compound, and the biphenyl compound has a structure represented by Formula I: 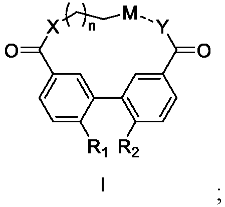
wherein R₁ and R₂ are each independently selected from any one of hydrogen, halogen, hydroxy, dimethylamino, cyano, nitro, C1-C8 (which, for example, may be C1, C2, C3, C4, C5, C6, C7 or C8) alkoxycarbonyl, C1-C8 (which, for example, may be C1, C2, C3, C4, C5, C6, C7 or C8) alkylcarbonyloxy, methylamino, methanesulfonyl, dimethylaminesulfonyl, amino, carboxyl, C1-C8 (which, for example, may be C1, C2, C3, C4, C5, C6, C7 or C8) alkyl, C1-C8 (which, for example, may be C1, C2, C3, C4, C5, C6, C7 or C8) alkoxy, C1-C8 (which, for example, may be C1, C2, C3, C4, C5, C6, C7 or C8) trihaloalkyl, C1-C8 (which, for example, may be C1, C2, C3, C4, C5, C6, C7 or C8) trihaloalkoxy, C1-C8 (which, for example, may be C1, C2, C3, C4, C5, C6, C7 or C8) alkoxymethyleneoxy, C1-C8 (which, for example, may be C1, C2, C3, C4, C5, C6, C7 or C8) methoxymethyleneoxy, cyclopentyloxy or cyclohexyloxy;
n is selected from 0, 1, 2, 3, or 4;
X is selected from CH₂, O or NH;
Y is selected from any one of CH₂, O, OH, NH, NH₂, NH(CH₂)_{n'}NH₂, NH(CH₂)_{n'}NHCOCF₃, OBn, C1-C8 (which, for example, may be C1, C2, C3, C4, C5, C6, C7 or C8) alkyl, C1-C8 (which, for example, may be C1, C2, C3, C4, C5, C6, C7 or C8) alkoxy, trihalomethylamino, dimethylamino, methylamino or trihaloalkoxy, wherein n' is selected from 1, 2, 3, 4 or 5;
M is selected from any one of CH₂, O, OH, NH, NH₂, trihalomethylamino, trihalomethylacyl, dimethylamino, methylamino, carboxyl, trihaloalkyl or C1-C8 alkoxymethyleneoxy; and M is joined to Y by a bond or M does not form a bond with Y.

In the present application, "trihalo" in C1-C8 trihaloalkyl or C1-C8 trihaloalkoxy means containing three halogens. C1-C8 trihaloalkyl includes F₃ C1-C8 alkyl, Br₃ C1-C8 alkyl, Cl₃ C1-C8 alkyl, I₃ C1-C8 alkyl, F₂Br C1-C8 alkyl, F₂Cl C1-C8 alkyl, F₂I C1-C8 alkyl, FBr₂ C1-C8 alkyl, FCl₂ C1-C8 alkyl, FI₂ C1-C8 alkyl, ClBr₂ C1-C8 alkyl, ICl₂ C1-C8 alkyl, and ClI₂ C1-C8 alkyl. C1-C8 trihaloalkoxy includes F₃ C1-C8 alkoxy, Br₃ C1-C8 alkoxy, Cl₃ C1-C8 alkoxy, I₃ C1-C8 alkoxy, F₂Br C1-C8 alkoxy, F₂Cl C1-C8 alkoxy, F₂I C1-C8 alkoxy, FBr₂ C1-C8 alkoxy, FCl₂ C1-C8 alkoxy, FI₂ C1-C8 alkyl, ClBr₂ C1-C8 alkoxy, ICl₂ C1-C8 alkoxy, and ClI₂ C1-C8 alkoxy.

Preferably, C1-C8 trihaloalkyl includes F₃ C1-C4 alkyl, Br₃ C1-C4 alkyl, Cl₃ C1-C4 alkyl, I₃ C1-C4 alkyl, F₂Br C1-C4 alkyl, F₂Cl C1-C4 alkyl, F₂I C1-C4 alkyl, FBr₂ C1-C4 alkyl, FCl₂ C1-C4 alkyl, FI₂ C1-C4 alkyl, ClBr₂ C1-C4 alkyl, ICl₂ C1-C4 alkyl, and ClI₂ C1-C4 alkyl; C1-C8 trihaloalkoxy includes F₃ C1-C4 alkoxy, Br₃ C1-C4 alkoxy, Cl₃ C1-C4 alkoxy, I₃ C1-C4 alkoxy, F₂Br C1-C4 alkoxy, F₂Cl C1-C4 alkoxy, F₂I C1-C4 alkoxy, FBr₂ C1-C4 alkoxy, FCl₂ C1-C4 alkoxy, FI₂ C1-C4 alkyl, ClBr₂ C1-C4 alkoxy, ICl₂ C1-C4 alkoxy, and ClI₂ C1-C4 alkoxy.

In the present application, "trihalo" in trihalomethylamino, trihalomethylacyl, and trihaloalkyl means containing three halogens.

In the present application, "C1-C8" in C1-C8 methoxymethyleneoxy means that the repeat unit of methylene is 1-8 (which, for example, may be 1, 2, 3, 4, 5, 6, 7 or 8).

In the present application, R1 and R2 are each independently selected from any one of hydrogen, fluorine, chlorine, bromine, hydroxy, dimethylamino, cyano, nitro, C1-C6 (which, for example, may be C1, C2, C3, C4, C5 or C6) alkoxycarbonyl, C1-C6 (which, for example, may be C1, C2, C3, C4, C5 or C6) alkylcarbonyloxy, methylamino, methanesulfonyl, dimethylaminesulfonyl, amino, carboxyl, C1-C6 (which, for example, may be C1, C2, C3, C4, C5 or C6) alkyl, C1-C7 (which, for example, may be C1, C2, C3, C4, C5, C6 or C7) alkoxy, C1-C6 (which, for example, may be C1, C2, C3, C4, C5 or C6) trihaloalkyl, C1-C7 (which, for example, may be C1, C2, C3, C4, C5, C6 or C7) trihaloalkoxy, C1-C6 (which, for example, may be C1, C2, C3, C4, C5 or C6) alkoxymethyleneoxy, C1-C6 (which, for example, may be C1, C2, C3, C4, C5 or C6) methoxymethyleneoxy, cyclopentyloxy or cyclohexyloxy;
n is selected from 1, 2, 3 or 4;
X is selected from CH₂, O or NH;
Y is selected from any one of CH₂, O, OH, NH, NH₂, NH(CH₂)_{n'}NH₂, NH(CH₂)_{n'}NHCOCF₃, OBn, C1-C6 (which, for example, may be C1, C2, C3, C4, C5 or C6) alkyl, C1-C6 (which, for example, may be C1, C2, C3, C4, C5 or C6) alkoxy, trihalomethylamino, dimethylamino, methylamino or trihaloalkoxy, wherein n' is selected from 2, 3, 4 or 5;
M is selected from any one of CH₂, O, OH, NH, NH₂, trihalomethylamino, trihalomethylacyl, dimethylamino, methylamino, carboxyl, trihaloalkyl or C1-C6 (which, for example, may be C1, C2, C3, C4, C5 or C6) alkoxymethyleneoxy; and
M is joined to Y by a bond or M does not form a bond with Y.

In the present application, R₁ and R₂ are each independently selected from any one of hydrogen, fluorine, chlorine, bromine, hydroxy, dimethylamino, cyano, nitro, C1-C4 (which, for example, may be C1, C2, C3 or C4) alkoxycarbonyl, C1-C4 (which, for example, may be C1, C2, C3 or C4) alkylcarbonyloxy, methylamino, methanesulfonyl, dimethylaminesulfonyl, amino, carboxyl, C1-C4 (which, for example, may be C1, C2, C3 or C4) alkyl, C1-C7 (which, for example, may be C1, C2, C3, C4, C5, C6 or C7) alkoxy, C1-C4 (which, for example, may be C1, C2, C3 or C4) trihaloalkyl, C1-C7 (which, for example, may be C1, C2, C3, C4, C5, C6 or C7) trihaloalkoxy, C1-C4 (which, for example, may be C1, C2, C3 or C4) alkoxymethyleneoxy, C1-C4 (which, for example, may be C1, C2, C3 or C4) methoxymethyleneoxy, cyclopentyloxy or cyclohexyloxy;
n is selected from 1, 2, 3 or 4;
X is selected from CH₂, O or NH;
Y is selected from any one of CH₂, O, OH, NH, NH₂, NH(CH₂)_{n'}NH₂, NH(CH₂)_{n'}NHCOCF₃, OBn, C1-C4 (which, for example, may be C1, C2, C3 or C4) alkyl, C1-C4 (which, for example, may be C1, C2, C3 or C4) alkoxy, trihalomethylamino, dimethylamino, methylamino or trihaloalkoxy, wherein n' is selected from 2, 3, 4 or 5;
M is selected from any one of CH₂, O, OH, NH, NH₂, trihalomethylamino, trihalomethylacyl, dimethylamino, methylamino, carboxyl, trihaloalkyl or C1-C4 alkoxymethyleneoxy; and M is joined to Y by a bond or M does not form a bond with Y.

In the present application, R₁ and R₂ are each independently selected from any one of hydrogen, fluorine, chlorine, bromine, hydroxy, dimethylamino, cyano, nitro, methoxycarbonyl, ethoxycarbonyl, methylcarbonyloxy, ethoxycarbonyl, methylamino, methanesulfonyl, dimethylaminesulfonyl, amino, carboxyl, methyl, ethyl, methoxy, ethoxy, propoxy, 1-butanyloxy, 1-pentyloxy, 1-hexanyloxy, 1-heptanyloxy, 2-pentyloxy, 2-hexanyloxy, trifluoromethyl, trifluoroethyl, trifluoromethanyloxy, methoxymethyleneoxy, methoxyethyloxy, methoxypropoxy, methoxybutyloxy, cyclopentyloxy or cyclohexyloxy;
n is selected from 1, 2, 3 or 4;
X is selected from CH₂, O or NH;
Y is selected from any one of CH₂, O, OH, NH, NH₂, NH(CH₂)_{n'}NH₂, NH(CH₂)_{n'}NHCOCF₃, OBn, methyl, ethyl, methoxy, ethoxy, trifluoromethylamino, trichloromethylamino, dimethylamino, methylamino or trifluoromethanyloxy, wherein n' is selected from 2, 3, 4 or 5;
M is selected from any one of CH₂, O, OH, NH, NH₂, trifluoromethylamino, trichloromethylamino, trifluoromethylacyl, trichloromethylacyl, dimethylamino, methylamino, carboxyl, trifluoromethyl or methoxymethyleneoxy; and
M is joined to Y by a bond or M does not form a bond with Y.

In the present application, the compound is selected from any one of the following structures represented by 1 to 20:

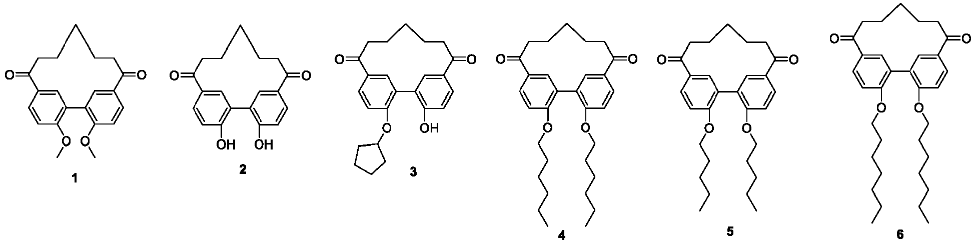

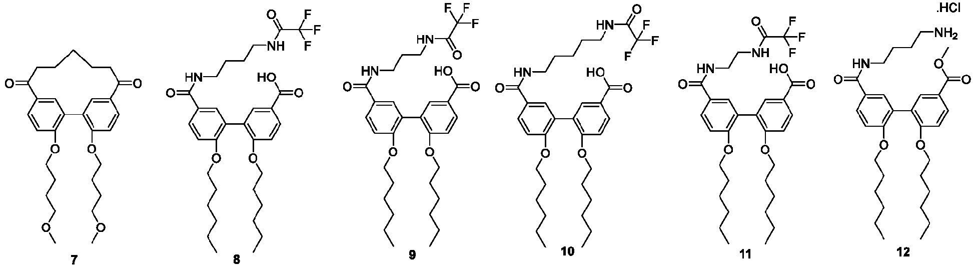

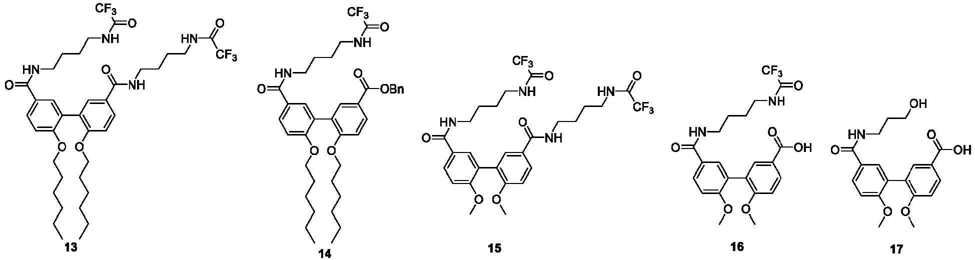

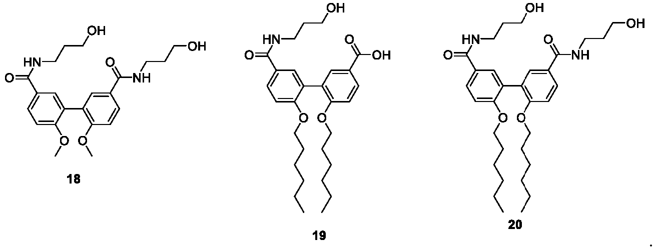

In a second aspect, the present application provides an isomer or a pharmaceutically acceptable salt of the biphenyl compound described in the first aspect.

Preferably, the pharmaceutically acceptable salt includes any one or a combination of at least two of a hydrochloride, a hydrobromide, a phosphate, a sulphate, a methanesulfonate, a p-toluenesulfonate, an acetate, a trifluoroacetate, a salicylate, an amino acid salt, a 2-O-β-D-Glucopyranosyl-L-ascorbic acid salt, a maleate, a tartrate, a fumarate, a citrate, a lactate, a sodium salt, a potassium salt, a calcium salt, a magnesium salt, a lithium salt, an ammonium salt, or a salt of an organic alkali capable of providing a physiologically acceptable cation.

Preferably, the salt of an organic alkali capable of providing a physiologically acceptable cation includes any one or a combination of at least two of a methylamine salt, a dimethylamine salt, a trimethylamine salt, a piperidine salt, a morpholine salt or a tris(2-hydroxyethyl)amine salt.

All salts in the present application may be prepared by conventional methods. In addition, during the preparation of solvates and salts of the compound of Formula (I), polycrystals or co-crystals may occur under different crystallization conditions.

In the present application, the term "halogen" refers to fluorine, chlorine, bromine or iodine.

The present application includes all possible stereoisomers as well as mixtures of two or more isomers.

In a third aspect, the present application provides a preparation method for the biphenyl compound described in the first aspect, and the preparation method includes the following steps:
synthesis method one:
(A) carrying out a nucleophilic addition reaction on a compound represented by Formula II, a compound represented by Formula III, and a compound represented by Formula IV to produce a compound represented by Formula V, where the reaction formula is as follows: 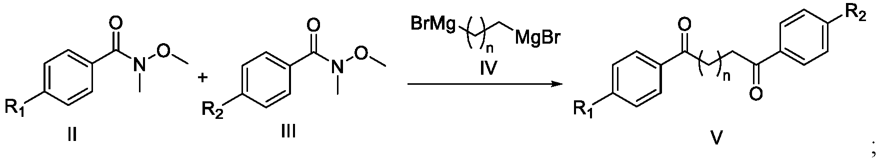 wherein the nucleophilic addition reaction is carried out in an organic solvent, and the organic solvent includes any one or a combination of at least two of tetrahydrofuran, diethyl ether, toluene or benzene; and the nucleophilic addition reaction is carried out at a temperature of 0 °C to 30 °C (which, for example, may be 0 °C, 5 °C, 10 °C, 15 °C, 20 °C, 25 °C, 30 °C, etc.) for a period of 1 h to 10 h (which, for example, may be 1 h, 2 h, 3 h, 4 h, 5 h, 6 h, 7 h, 8 h, 9 h, 10 h, etc.);
(B) carrying out an iodination reaction on the compound represented by Formula V and iodine to produce a compound represented by Formula VI, where the reaction formula is as follows: 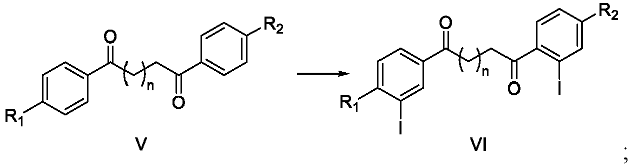 wherein the iodination reaction is carried out in the presence of a fluorine reagent, and the iodination reaction is carried out at a temperature of 0 °C to 30 °C (which, for example, may be 0 °C, 5 °C, 10 °C, 15 °C, 20 °C, 25 °C, 30 °C, etc.) for a period of 1 h to 100 h (which, for example, may be 1 h, 10 h, 20 h, 30 h, 40 h, 50 h, 60 h, 70 h, 80 h, 90 h, 100 h, etc.);
(C) carrying out a coupling reaction on the compound represented by Formula VI in an alkaline condition in the presence of zero-valent palladium to produce a compound represented by Formula I, where the reaction formula is as follows: 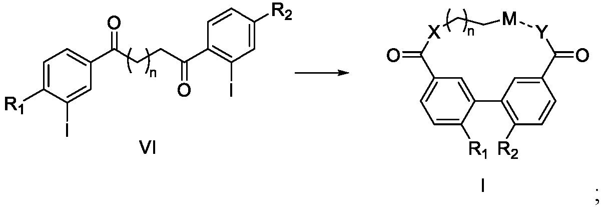
   wherein the coupling reaction is carried out in the presence of an organic alkali and/or an inorganic alkali, the organic alkali includes any one or a combination of at least two of pyridine, piperidine, diisopropylamine, ethylenediamine, triethylamine or *N,N-*diisopropylethylamine, and the inorganic alkali includes any one or a combination of at least two of sodium hydroxide, potassium hydroxide, lithium hydroxide, cesium carbonate or potassium carbonate; the coupling reaction is carried out in a solvent, and the solvent includes any one or a combination of at least two of dimethylsulfoxide, N,N-dimethylformamide or dioxane; and the coupling reaction is carried out at a temperature of 30 °C to 120 °C (which, for example, may be 30 °C, 50 °C, 70 °C, 90 °C, 100 °C, 110 °C, 120 °C, etc.) for a period of 3 h-20 h (which, for example, may be 3 h, 5 h, 7 h, 9 h, 11 h, 13 h, 15 h, 17 h, 20 h, etc.);
   wherein R₁ and R₂ are each independently selected from any one of hydrogen, halogen, dimethylamino, cyano, nitro, C1-C8 alkoxycarbonyl, methylamino, methanesulfonyl, dimethylaminesulfonyl, amino, carboxyl, C1-C8 alkyl or C1-C8 trihaloalkyl, n is selected from 0, 1, 2, 3 or 4, and X, Y, and M are each independently selected from CH₂;
   or, synthesis method two:
(D) carrying out a nucleophilic addition reaction on a compound represented by Formula II' and a compound represented by Formula III' to produce a compound represented by Formula IV', or carrying out a nucleophilic addition reaction on a compound represented by Formula II' and a compound represented by Formula II to produce a compound represented by Formula IV", or carrying out a nucleophilic addition reaction on a compound represented by Formula II' and a compound represented by Formula III to produce a compound represented by Formula IV"', where the reaction formula is as follows: 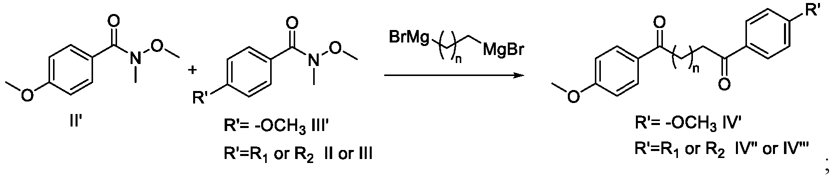 wherein the nucleophilic addition reaction is carried out in an organic solvent, and the organic solvent includes any one or a combination of at least two of tetrahydrofuran, diethyl ether, toluene or benzene; and the nucleophilic addition reaction is carried out at a temperature of 0 °C to 30 °C (which, for example, may be 0 °C, 5 °C, 10 °C, 15 °C, 20 °C, 25 °C, 30 °C, etc.) for a period of 1 h to 10 h (which, for example, may be 1 h, 3 h, 5 h, 7 h, 9 h, 10 h, etc.);
(E) carrying out an iodination reaction on the compound represented by Formula IV' to produce a compound represented by Formula V', or carrying out an iodination reaction on the compound represented by Formula IV" to produce a compound represented by Formula V", or carrying out an iodination reaction on the compound represented by Formula IV‴ to produce a compound represented by Formula V‴, where the reaction formula is as follows: 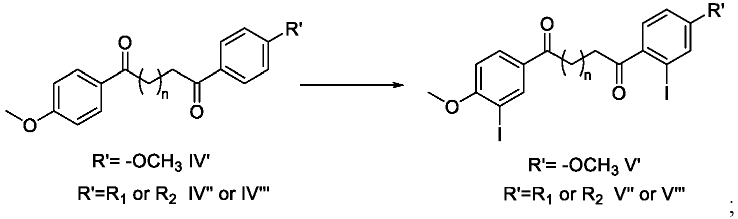 wherein the iodination reaction is carried out in the presence of a fluorine reagent; and the iodination reaction is carried out at a temperature of 0 °C to 30 °C (which, for example, may be 0 °C, 5 °C, 10 °C, 15 °C, 20 °C, 25 °C, 30 °C, etc.) for a period of 1 h to 100 h (which, for example, may be 1 h, 10 h, 20 h, 30 h, 40 h, 50 h, 60 h, 70 h, 80 h, 90 h, 100 h, etc.);
(F) carrying out a coupling reaction on the compound represented by Formula V' in an alkaline condition in the presence of zero-valent palladium, and carrying out hydrolysis in a Lewis acid condition to produce a compound represented by formula VI'; or carrying out a coupling reaction on the compound represented by Formula V" in an alkaline condition in the presence of zero-valent palladium, and carrying out hydrolysis in a Lewis acid condition to produce a compound represented by formula VI"; or carrying out a coupling reaction on the compound represented by Formula V‴ in an alkaline condition in the presence of zero-valent palladium, and carrying out hydrolysis in a Lewis acid condition to produce a compound represented by formula VI"'; where the reaction formula is as follows: 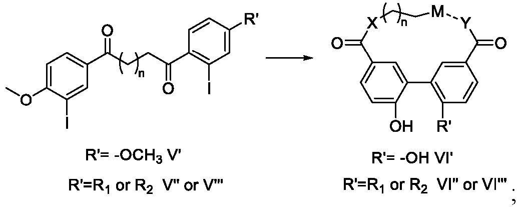
   wherein the coupling reaction is carried out in the presence of an organic alkali and/or an inorganic alkali, the organic alkali includes any one or a combination of at least two of pyridine, piperidine, diisopropylamine, ethylenediamine, triethylamine or *N,N-*diisopropylethylamine, and the inorganic alkali includes any one or a combination of at least two of sodium hydroxide, potassium hydroxide, lithium hydroxide, cesium carbonate or potassium carbonate; the coupling reaction is carried out in a solvent, and the solvent includes any one or a combination of at least two of dimethylsulfoxide, *N*,*N*-dimethylformamide or dioxane; and the coupling reaction is carried out at a temperature of 30 °C to 120 °C (which, for example, may be 30 °C, 50 °C, 70 °C, 90 °C, 100 °C, 110 °C, 120 °C, etc.) for a period of 3 h to 20 h (which, for example, may be 3 h, 5 h, 7 h, 9 h, 11 h, 13 h, 15 h, 17 h, 20 h, etc.); and
   the Lewis acid includes boron tribromide and/or aluminum trichloride; the hydrolysis is carried out at a temperature of -70 °C to -40 °C (which, for example, may be -70 °C, -60 °C, - 50 °C, -40 °C, etc.) for a period of 1 to 10 h (which, for example, may be 1 h, 3 h, 5 h, 7 h, 9 h, 10 h, etc.);
(G) carrying out an alkylation reaction on the compound represented by Formula VI' and an alkyl halide compound to produce a compound represented by Formula I; or carrying out an alkylation reaction on the compound represented by Formula VI" and an alkyl halide compound to produce a compound represented by Formula I; or carrying out an alkylation reaction on the compound represented by Formula VI‴ and an alkyl halide compound to produce a compound represented by Formula I; wherein the alkyl halide compound includes an alkyl bromide or an alkyl chloride, and the reaction formula is as follows: 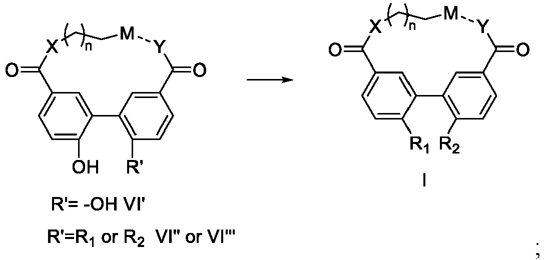
   wherein the alkylation reaction is carried out in the presence of an organic alkali and/or an inorganic alkali, the organic alkali includes any one or a combination of at least two of pyridine, piperidine, diisopropylamine, ethylenediamine, triethylamine or *N,N-*diisopropylethylamine, and the inorganic alkali includes any one or a combination of at least two of sodium hydroxide, potassium hydroxide, lithium hydroxide, cesium carbonate or potassium carbonate; and the alkylation reaction is carried out at a temperature of 0 °C to 30 °C (which, for example, may be 0 °C, 5°C, 10 °C, 15 °C, 20 °C, 25 °C, 30 °C, etc.) for a period of 1 h to 10 h (which, for example, may be 1 h, 3 h, 5 h, 7 h, 9 h, 10 h, etc.);
   wherein R₁ and R₂ are each independently selected from any one of hydrogen, halogen, hydroxy, dimethylamino, cyano, nitro, C1-C8 alkoxycarbonyl, C1-C8 alkylcarbonyloxy, methylamino, methanesulfonyl, dimethylaminesulfonyl, amino, carboxyl, C1-C8 alkyl, C1-C8 alkoxy, C1-C8 trihaloalkyl, C1-C8 trihaloalkoxy, C1-C8 alkoxymethyleneoxy, C1-C8 methoxymethyleneoxy, cyclopentyloxy or cyclohexyloxy, n is selected from 0, 1, 2, 3 or 4, and X, Y, and M are each independently selected from CH₂;
   or, synthesis method three:
(H) carrying out a hydrolysis reaction on a compound represented by Formula VII and alkali to produce a compound represented by Formula VIII; wherein the alkali includes an organic alkali and/or an inorganic alkali, and the reaction formula is as follows: 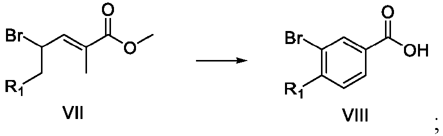 wherein the organic alkali includes any one or a combination of at least two of pyridine, piperidine, diisopropylamine, ethylenediamine, triethylamine or *N,N-*diisopropylethylamine, and the inorganic alkali includes any one or a combination of at least two of sodium hydroxide, potassium hydroxide, lithium hydroxide, cesium carbonate or potassium carbonate; the hydrolysis reaction is carried out at a temperature of 10 °C to 100 °C (which, for example, may be 10 °C, 30 °C, 50 °C, 70 °C, 90 °C, 100 °C, etc.) for a period of 1 h to 40 h (which, for example, may be 1 h, 3 h, 5 h, 7 h, 9 h, 11 h, 13 h, 15 h, 17 h, 20 h, 25 h, 30 h, 35 h, 40 h, etc.); and the hydrolysis reaction is carried out in the presence of a solvent, and the solvent includes any one or a combination of at least two of tetrahydrofuran, methanol, ethanol or water;
(I) carrying out a condensation reaction on the compound represented by Formula VIII and a compound represented by Formula IX in an alkaline condition to produce a compound represented by Formula X, where the reaction formula is as follows: 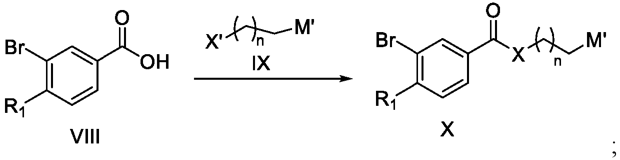 wherein the condensation reaction is carried out in the presence of an organic alkali and/or an inorganic alkali, the organic alkali includes any one or a combination of at least two of pyridine, piperidine, diisopropylamine, ethylenediamine, triethylamine or *N,N-*diisopropylethylamine, and the inorganic alkali includes any one or a combination of at least two of sodium hydroxide, potassium hydroxide, lithium hydroxide, cesium carbonate or potassium carbonate; the condensation reaction is carried out at a temperature of 10 °C to 30 °C (which, for example, may be 10 °C, 15 °C, 20 °C, 25 °C, 30 °C, etc.) for a period of 1 h to 40 h (which, for example, may be 1 h, 3 h, 5 h, 7 h, 9 h, 11 h, 13 h, 15 h, 17 h, 20 h, 25 h, 30 h, 35 h, 40 h, etc.); the condensation reaction is carried out in the presence of a solvent, and the solvent includes any one or a combination of at least two of dichloromethane, tetrahydrofuran or *N,N-*dimethylformamide; and the condensation reaction is carried out in the presence of a condensing agent, the condensing agent includes any one or a combination of at least two of carbodiimide hydrochloride (EDCI), dicyclohexylcarbodiimide (DCC), 2-(7-azabenzotriazole)-*N*,*N*,*N*',*N*'-tetramethyluronium hexafluorophosphate (HATU) or 1-hydroxybenzotriazole (HOBt);
(J) carrying out a coupling reaction on the compound represented by Formula X to produce a compound represented by Formula XI, where the reaction formula is as follows: 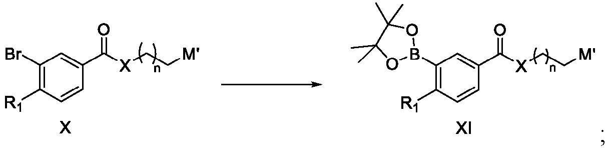
   wherein the coupling reaction is carried out in the presence of an organic alkali and/or an inorganic alkali, the organic alkali includes any one or a combination of at least two of pyridine, piperidine, diisopropylamine, ethylenediamine, triethylamine or *N*,*N-*diisopropylethylamine, and the inorganic alkali includes any one or a combination of at least two of sodium hydroxide, potassium hydroxide, lithium hydroxide, cesium carbonate or potassium carbonate; the coupling reaction is carried out at a temperature of 30 °C to 120 °C (which, for example, may be 30 °C, 50 °C, 70 °C, 90 °C, 100 °C, 110 °C, 120 °C, etc.) for a period of 3 h to 20 h (which, for example, may be 3 h, 5 h, 7 h, 9 h, 11 h, 13 h, 15 h, 17 h, 20 h, etc.); and the coupling reaction is carried out in the presence of a solvent, and the solvent includes any one or a combination of at least two of dimethylsulfoxide, *N*,*N*-dimethylformamide, dioxane or ethylene glycol dimethyl ether;
   the compound represented by Formula X undergoes a coupling reaction with bis(pinacolato)diboron;
(K) carrying out a coupling reaction the compound represented by Formula XI and a compound represented by Formula XII in an alkaline condition in the presence of zero-valent palladium to produce a compound represented by Formula I; or carrying out a coupling reaction on the compound represented by Formula XI and a compound represented by Formula XII in an alkaline condition in the presence of zero-valent palladium, carrying out hydrolysis in the presence of an organic alkali or an inorganic alkali after the reaction, stirring the resulting product in a hydrochloric acid or a trifluoroacetic acid to obtain an amino hydrochloride salt, separating, and reacting with an acyl reagent to obtain a compound represented by Formula I; where the reaction formula is as follows: 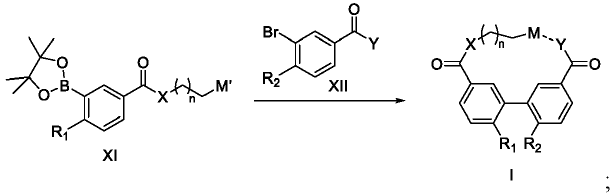
   wherein the coupling reaction is carried out in the presence of an organic alkali and/or an inorganic alkali, the organic alkali includes any one or a combination of at least two of pyridine, piperidine, diisopropylamine, ethylenediamine, triethylamine or *N*,*N-*diisopropylethylamine, and the inorganic alkali includes any one or a combination of at least two of sodium hydroxide, potassium hydroxide, lithium hydroxide, cesium carbonate or potassium carbonate; the coupling reaction is carried out at a temperature of 30 °C to 120 °C (which, for example, may be 30 °C, 50 °C, 70 °C, 90 °C, 100 °C, 110 °C, 120 °C, etc.) for a period of 3 h to 20 h (which, for example, may be 3 h, 5 h, 7 h, 9 h, 11 h, 13 h, 15 h, 17 h, 20 h, etc.); and the coupling reaction is carried out in the presence of a solvent, and the solvent includes any one or a combination of at least two of dimethylsulfoxide, *N*,*N*-dimethylformamide, dioxane or ethylene glycol dimethyl ether;
   the concentration of the hydrochloric acid or the trifluoroacetic acid is 0.8 N to 1.2 N (which, for example, may be 0.8 N, 0.9 N, 1 N, 1.1 N, 1.2 N, etc.);
   wherein R₁ and R₂ are each independently selected from any one of hydrogen, halogen, hydroxy, dimethylamino, cyano, nitro, C1-C8 alkoxycarbonyl, C1-C8 alkylcarbonyloxy, methylamino, methanesulfonyl, dimethylaminesulfonyl, amino, carboxyl, C1-C8 alkyl, C1-C8 alkoxy, C1-C8 trihaloalkyl, C1-C8 trihaloalkoxy, C1-C8 alkoxymethyleneoxy, C1-C8 methoxymethyleneoxy, cyclopentyloxy or cyclohexyloxy, n is selected from 0, 1, 2, 3 or 4, X is selected from CH₂, O or NH, X' is selected from OH or NH₂, and Y is selected from any one of CH₂, O, OH, NH, NH₂, NH(CH₂)_{n'}NH₂, NH(CH₂)_{n'}NHCOCF₃, OBn, C₁₋₈ alkyl, C₁₋₈ alkoxy, trihalomethylamino, dimethylamino, methylamino or trihaloalkoxy, wherein n' is selected from 1, 2, 3, 4 or 5; and
   M is selected from any one of CH₂, O, OH, NH, NH₂, trihalomethylamino, trihalomethylacyl, dimethylamino, methylamino, carboxyl, trihaloalkyl or C1-C8 alkoxymethyleneoxy, and M' is selected from any one of OH, NH₂, trihalomethylamino, trihalomethylacyl, dimethylamino, methylamino, carboxyl, trihaloalkyl or C1-C8 alkoxymethyleneoxy.

In a fourth aspect, the present application provides a pharmaceutical composition, and the pharmaceutical composition includes an active ingredient and a pharmacodynamically acceptable carrier, wherein the active ingredient includes the biphenyl compound described in the first aspect or the isomer or the pharmaceutically acceptable salt of the biphenyl compound described in the second aspect.

Preferably, a mass percentage of the active ingredient in the pharmaceutical composition is 0.1% to 95%.

The pharmaceutical composition provided by the present application may be prepared according to methods well known in the art and may be prepared in combination with one or more pharmaceutically acceptable solid or liquid excipients and/or adjuvants into any dosage form suitable for humans or animals with the active ingredient.

The biphenyl compound provided in the first aspect, the isomer or the pharmaceutically acceptable salt of the biphenyl compound provided in the second aspect or the pharmaceutical composition provided in the fourth aspect in the present application may be administered in a unit dose form, and the route of administration may be enteral or parenteral, such as oral administration, intravenous injection, intramuscular injection, subcutaneous injection, nasal administration, oromucosal administration, ophthalmic administration, administration through lungs and the respiratory tract, dermal administration, intravaginal administration, rectal administration, etc.

The dosage form may be a liquid dosage form, a solid dosage form or a semi-solid dosage form. The liquid dosage form may be solutions (including true solutions and colloidal solutions), emulsions (including o/w emulsions, w/o emulsions, and multiple emulsions), suspensions, injections (including aqueous injections, powder injections, and infusions), eye drops, nasal drops, lotions, and liniments. The solid dosage form may be tablets (including conventional tablets, enteric-coated tablets, buccal tablets, dispersible tablets, chewable tablets, effervescent tablets, and orally disintegrating tablet), capsules (including hard capsules, soft capsules, and enteric-coated capsules), granules, dispersions, micropellets, drops, suppositories, films, patches, (powder) aerosols, and sprays. The semi-solid dosage form may be ointments, gels, and pastes.

The biphenyl compound provided in the first aspect, the isomer or the pharmaceutically acceptable salt of the biphenyl compound provided in the second aspect or the pharmaceutical composition provided in the fourth aspect in the present application may be prepared into conventional formulations or may be prepared into sustained release formulations, controlled release formulations, targeting formulations, and various particulate delivery systems.

To prepare the biphenyl compound provided in the first aspect, the isomer or the pharmaceutically acceptable salt of the biphenyl compound provided in the second aspect or the pharmaceutical composition provided in the fourth aspect in the present application into tablets, various excipients well known in the art, including diluents, binding agents, wetting agents, disintegrating agents, lubricants, and glidants, may be used. The diluent may be starch, dextrin, sucrose, glucose, lactose, mannitol, sorbitol, xylitol, microcrystalline cellulose, calcium sulphate, calcium hydrogen phosphate, and calcium carbonate. The wetting agent may be water, ethanol, and isopropanol. The binding agent may be starch slurry, dextrin, molasses, honey, glucose solution, microcrystalline cellulose, mucilago acaciae, gelatine, sodium carboxymethyl cellulose, methylcellulose, hydroxypropyl methylcellulose, ethylcellulose, acrylic resin, carbomer, polyvinylpyrrolidone, and polyethylene glycol. The disintegrating agent may be dry starch, microcrystalline cellulose, low-substituted hydroxypropyl cellulose, cross-linked polyvinylpyrrolidone, cross-linked sodium carboxymethylcellulose, sodium carboxymethyl starch, sodium bicarbonate with citric acid, polyoxyethylene sorbitol fatty acid ester, and sodium dodecyl sulfate. The lubricant and the glidant may be talcum powder, silicon dioxide, stearate, tartaric acid, liquid paraffin, and polyethylene glycol.

The tablets may further be prepared into coated tablets, such as sugar-coated tablets, film-coated tablets, enteric-coated tablets, or bilayer tablets and multilayer tablets.

To prepare the biphenyl compound provided in the first aspect, the isomer or the pharmaceutically acceptable salt of the biphenyl compound provided in the second aspect or the pharmaceutical composition provided in the fourth aspect in the present application into capsules, the biphenyl compound provided in the first aspect, the isomer or the pharmaceutically acceptable salt of the biphenyl compound provided in the second aspect or the pharmaceutical composition provided in the fourth aspect may be mixed with a diluent and a glidant, and the resulting mixture is directly loaded in a hard capsule or a soft capsule. The compound of the present application serving as the effective constituent may also be mixed with a diluent, a binding agent, and a disintegrating agent and prepared into granules or micropellets, and the resulting granules or micropellets are loaded in a hard capsule or a soft capsule. The diluents, binding agents, wetting agents, disintegrating agents, and glidants used in the preparation of tablets may also be used in the preparation of capsules.

To prepare the biphenyl compound provided in the first aspect, the isomer or the pharmaceutically acceptable salt of the biphenyl compound provided in the second aspect or the pharmaceutical composition provided in the fourth aspect in the present application into injections, water, ethanol, isopropanol, propylene glycol or a mixture thereof may be used as a solvent, and appropriate quantities of solubilizer, cosolvent, pH adjusting agent, and osmotic pressure regulator, which are commonly used in the art, may be added. The solubilizer or cosolvent may be poloxamer, lecithin, and hydroxypropyl-beta-cyclodextrin. The pH adjusting agent may be phosphate, acetate, hydrochloric acid, and sodium hydroxide. The osmotic pressure regulator may be sodium chloride, mannitol, glucose, phosphate, and acetate. If a lyophilized powder injection is to be prepared, mannitol, glucose, and the like may also be added as a support agent.

In addition, colorants, preservatives, spices, corrigent or other additives may also be added to the pharmaceutical preparation, if required.

To achieve the medicinal purpose and enhance the therapeutic effect, the drug or the pharmaceutical composition of the present application may be administered by any well-known method of administration.

In a fifth aspect, the present provides the use of the biphenyl compound described in the first aspect, the isomer or the pharmaceutically acceptable salt of the biphenyl compound described in the second aspect or the pharmaceutical composition described in the fourth aspect in the preparation of a drug for preventing and/or treating a tumor, an autoimmune disease, an inflammatory disease, a neurodegenerative disease or a metabolic disease drug or an anti-aging drug.

The biphenyl compound provided in the first aspect, the isomer or the pharmaceutically acceptable salt of the biphenyl compound provided in the second aspect or the pharmaceutical composition provided in the fourth aspect in the present application acts on animals, preferably on mammals, and further preferably on humans.

In the present application, the tumor is selected from any one or a combination of at least two of glioma, melanoma, gastric cancer, lung cancer, breast cancer, kidney cancer, liver cancer, oral epithelial carcinoma, head and neck tumor, cervical cancer, ovarian cancer, pancreatic cancer, prostate cancer, colon cancer, rectal adenocarcinoma, leukemia or lymphoma.

Preferably, the autoimmune disease includes any one or a combination of at least two of rheumatoid arthritis, systemic lupus erythematosus, ulcerative colitis, psoriasis, dermatitis or amyotrophic lateral sclerosis.

Preferably, the inflammatory disease includes any one or a combination of at least two of polyarteritis, phlebitis or reflux esophagitis.

Preferably, the neurodegenerative disease includes senile dementia and/or Parkinson's disease.

Preferably, the metabolic disease includes diabetes, hyperuricemia or gout.

The dosage of administration of the biphenyl compound provided in the first aspect, the isomer or the pharmaceutically acceptable salt of the biphenyl compound provided in the second aspect or the pharmaceutical composition provided in the fourth aspect in the present application may vary widely, depending on the nature and severity of the disease to be prevented or treated, the individual condition of the patient or the animal, the route of administration, the dosage form, and the like. Generally, a suitable dosage per day ranges from 0.001 mg/Kg body weight to 150 mg/Kg body weight, preferably from 0.1 mg/Kg body weight to 100 mg/Kg body weight, more preferably from 1 mg/Kg body weight to 70 mg/Kg body weight, and most preferably 2 mg/Kg body weight to 30 mg/Kg body weight. The above dosage may be administered as a single dosage unit or divided into several dosage units, depending on the physician's clinical experience and the dosage regimen of other therapeutic means used together.

The biphenyl compound provided in the first aspect, the isomer or the pharmaceutically acceptable salt of the biphenyl compound provided in the second aspect or the pharmaceutical composition provided in the fourth aspect in the present application may be used alone or in combination with other therapeutic drugs or drugs for symptomatic treatment. When the biphenyl compound provided in the first aspect, the isomer or the pharmaceutically acceptable salt of the biphenyl compound provided in the second aspect or the pharmaceutical composition provided in the fourth aspect in the present application has a synergistic effect with other therapeutic drugs, the dosage thereof may be adjusted according to the actual situation.

Compared with the prior art, the present application has the beneficial effects described below.

The compound provided by the present application is a new biphenyl derivative. Such a compound has relatively good binding to multiple target proteins, has relatively good bioavailability, and can be used for preparing drugs for preventing or treating tumors, autoimmune diseases, inflammatory diseases, and neurodegenerative diseases or anti-aging drugs, wherein the tumor is glioma, melanoma, gastric cancer, lung cancer, breast cancer, kidney cancer, liver cancer, oral epithelial carcinoma, head and neck tumor, cervical cancer, ovarian cancer, pancreatic cancer, prostate cancer, colon cancer, rectal adenocarcinoma, leukemia or lymphoma.

### BRIEF DESCRIPTION OF DRAWINGS

FIG. 1 is an assay diagram of the interaction between the Human LAG3 protein and Compound 4.
FIG. 2 is an assay diagram of the interaction between the Mouse LAG3 protein and Compound 4.
FIG. 3 is an assay diagram of the interaction between the Human LAG3 protein and Compound 8.
FIG. 4 is an assay diagram of the interaction between the Mouse LAG3 protein and Compound 8.
FIG. 5 is an assay diagram of the interaction between the Human LAG3 protein and Compound 9.
FIG. 6 is an assay diagram of the interaction between the Mouse LAG3 protein and Compound 9.
FIG. 7 is an assay diagram of the interaction between the Human FGL1 protein and Compound 4.
FIG. 8 is an assay diagram of the interaction between the Human FGL1 protein and Compound 8.
FIG. 9 is an assay diagram of the interaction between the Human FGL1 protein and Compound 9.

### DETAILED DESCRIPTION

Technical solutions of the present application will be further described below through specific examples. Those skilled in the art are to understand that the examples set forth below are used for a better understanding of the present application and are not to be construed as specific limitations on the present application.

### Preparation Example 1

This preparation example provides Compound 21, and the method for preparing Compound 21 includes the following steps.

### Step A or D:

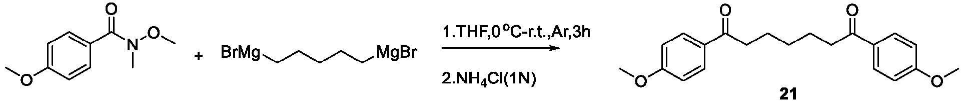

Pentane-1,5-bis(magnesium bromide) (0.5 M) (100 mL) was added dropwise to a tetrahydrofuran solution (100 mL) of *N*,4-dimethoxy-N-methylbenzamide (19.5 g, 100 mmol) in at 0 °C under argon protection. The reaction solution was stirred at room temperature for 3 h and quenched by dropwise addition of saturated ammonium chloride solution (100 mL). The reaction solution was extracted with ethyl acetate (50 mL × 3). The organic phases were combined, washed with saturated brine, dried with anhydrous sodium sulfate, and filtered. The resulting solution was concentrated under reduced pressure and purified by silica gel column chromatography (dichloromethane:ethyl acetate = 10: 1) to isolate a white solid **21** (8.4 g) (with a yield of 50%).

¹H NMR (400 MHz, CDCl₃) δ 7.97 - 7.90 (m, 4H), 6.96 - 6.90 (m, 4H), 3.87 (d, *J* = 1.9 Hz, 6H), 2.97 - 2.87 (m, 4H), 1.79 (dt, *J =* 20.7, 7.5 Hz, 4H), 1.53 - 1.41 (m, 2H).

### Preparation Example 2

This preparation example provides Compound 22, and the method for preparing Compound 22 includes the following steps.

### Step B or E:

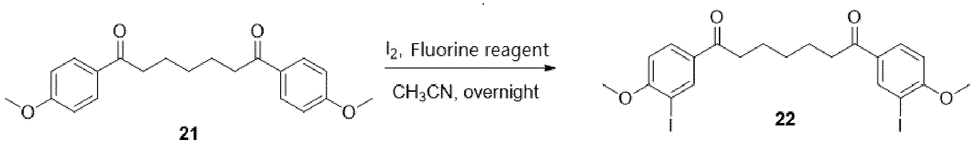

1,7-2 (4-methoxyphenyl)heptane-1,7-dione (23.84 g, 70 mmol) and a fluorine reagent (24.83 g, 70 mmol) were dissolved in acetonitrile (500 mL) at room temperature, and iodine (17.8 g, 70 mmol) was added to the solution. The solution was stirred at room temperature for three days. The solid obtained by filtering the solution was washed with water and ethanol, respectively, to give a white solid (30 g, with a yield of 70%).

¹H NMR (400 MHz, CDCl₃) δ 8.39 (d, *J* = 2.1 Hz, 2H), 7.95 (dd, *J* = 8.6, 2.2 Hz, 2H), 6.84 (d, *J =* 8.6 Hz, 2H), 3.95 (s, 6H), 2.92 (t, *J =* 7.3 Hz, 4H), 1.78 (dt, *J =* 15.0, 7.4 Hz, 4H), 1.47 (tt, *J =* 9.6, 6.4 Hz, 2H).

### Preparation Example 3

This preparation example provides Compound 24, and the method for preparing Compound 24 includes the following steps.

### Step H:

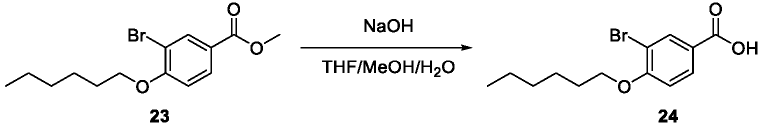

Methyl 3-bromo-4-hexyloxybenzoate **23** (2 g, 6.35 mmol) and sodium hydroxide (0.51 g, 12.7 mmol) were added to a mixed solution of tetrahydrofuran/methanol/water (8 mL/4 mL/6 mL), respectively, and stirred at room temperature for 16 h. The reaction solution was concentrated under reduced pressure, water (30 mL) was added, the pH of the reaction solution was adjusted to 5 with hydrochloric acid, and the reaction solution was filtered to give a white solid (1.55 g) (with a yield of 81%).

¹H NMR (400 MHz, MeOD-d4) δ 8.15 (d, *J =* 2.1 Hz, 1H), 7.96 (dd, *J =* 8.6, 2.1 Hz, 1H), 7.07 (d, *J =* 8.7 Hz, 1H), 4.12 (t, *J =* 6.3 Hz, 2H), 1.83 (dq, *J =* 12.5, 6.3 Hz, 2H), 1.62 - 1.47 (m, 2H), 1.44 - 1.29 (m, 4H), 1.01 - 0.85 (m, 3H).

### Preparation Example 4

This preparation example provides Compound 25, and the method for preparing Compound 25 includes the following steps.

### Step I:

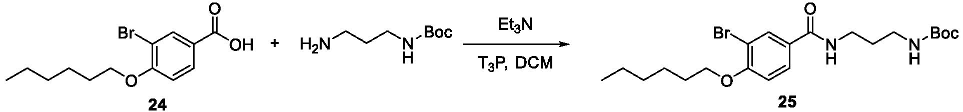

3-bromo-4-hexyloxybenzoic acid **24** (0.77g, 2.57 mmol), mono-Boc-propanediamine (0.58 g, 3.34 mmol), triethylamine (1.43 mL, 10.27 mmol), and propylphosphonic anhydride (30%) (4.9 g, 7.7 mmol) were added to anhydrous dichloromethane (15 mL), respectively, and stirred at room temperature for 16 h. Water (30 mL) was added to the reaction solution, and the reaction solution was extracted with dichloromethane (30 mL × 3) and backwashed with saturated sodium chloride solution. The organic phases were dried with anhydrous sodium sulfate and concentrated. The resulting crude product was purified by column chromatography (dichloromethane:methanol = 100:1 → 50:1) to isolate a yellow oil **25** (990 mg) (with a yield of 84%).

### Preparation Example 5

This preparation example provides Compound 26, and the method for preparing Compound 26 includes the following steps.

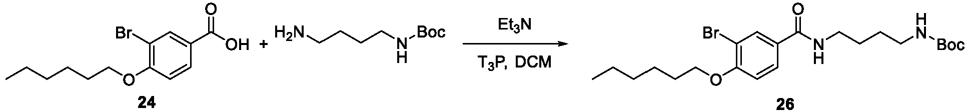

3-bromo-4-hexyloxybenzoic acid **24** (0.77 g, 2.57 mmol), mono-Boc-butanediamine (0.63 g, 3.34 mmol), triethylamine (1.43 mL, 10.27 mmol), and propylphosphonic anhydride (30%) (4.9 g, 7.7 mmol) were added to anhydrous dichloromethane (15 mL), respectively, and stirred at room temperature for 16 h. Water (30 mL) was added to the reaction solution, and the reaction solution was extracted with dichloromethane (30 mL × 3) and backwashed with saturated sodium chloride solution. The organic phases were dried with anhydrous sodium sulfate and concentrated. The resulting crude product was purified by column chromatography (dichloromethane:methanol = 100:1 → 50:1) to isolate a brown solid (1.1 g) (with a yield of 91%).

### Preparation Example 6

This preparation example provides Compound 27, and the method for preparing Compound 27 includes the following steps.

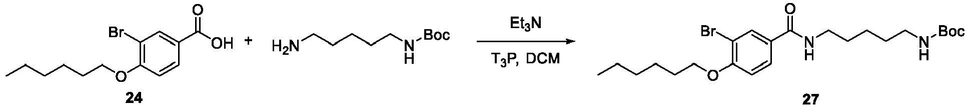

3-bromo-4-hexyloxybenzoic acid **24** (0.9 g, 3 mmol), mono-Boc-pentanediamine (0.8 g, 4 mmol), triethylamine (1.39 mL, 10 mmol), and propylphosphonic anhydride (30%) (5.7 g, 9 mmol) were added to anhydrous dichloromethane (30 mL), respectively, and stirred at room temperature for 16 h. Water (30 mL) was added to the reaction solution, and the reaction solution was extracted with dichloromethane (30 mL × 3) and backwashed with saturated sodium chloride solution. The organic phases were dried with anhydrous sodium sulfate and concentrated. The resulting crude product was purified by column chromatography (dichloromethane:methanol = 100:1 → 50:1) to isolate a white solid (1.22 g) (with a yield of 84%).

### Preparation Example 7

This preparation example provides Compound 28, and the method for preparing Compound 28 includes the following steps.

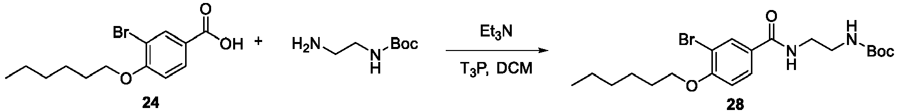

3-bromo-4-hexyloxybenzoic acid (0.9 g, 3 mmol), mono-Boc-pentanediamine (0.64 g, 4 mmol), triethylamine (1.39 mL, 10 mmol), and propylphosphonic anhydride (30%) (5.7g, 9 mmol) were added to anhydrous dichloromethane (30 mL), respectively, and stirred at room temperature for 16 h. Water (30 mL) was added to the reaction solution, and the reaction solution was extracted with dichloromethane (30 mL × 3) and backwashed with saturated sodium chloride solution. The organic phases were dried with anhydrous sodium sulfate and concentrated. The resulting crude product was purified by column chromatography (dichloromethane:methanol = 100:1 → 50:1) to isolate a white solid (1.07 g) (with a yield of 81%).

### Preparation Example 8

This preparation example provides Compound 29, and the method for preparing Compound 29 includes the following steps.

### Step J:

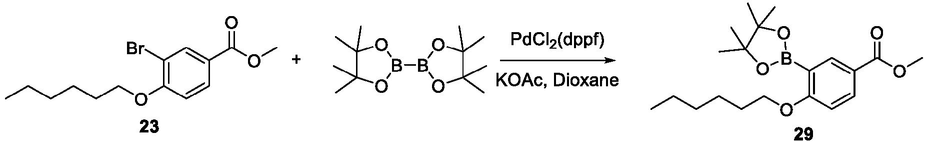

Methyl 3-bromo-4-hexyloxybenzoate **23** (2.5 g, 7.94 mmol), bis(pinacolato)diboron (2.42 g, 9.52 mmol), and potassium acetate (2.34 g, 23.8 mmol) were added to 1,4-dioxane (50 mL), respectively. The reaction solution was degassed with argon, 1,1'-bisdiphenylphosphinoferrocene palladium dichloride (0.58 g, 0.79 mmol) was added, and the reaction solution was stirred at 90 °C for 16 h. Water (50 mL) was added to the reaction solution, and the reaction solution was extracted with ethyl acetate (50 mL × 3) and backwashed with saturated sodium chloride solution. The organic phases were dried with anhydrous sodium sulfate and concentrated. The resulting crude product was purified by column chromatography (ethyl acetate:petroleum ether = 1:80 → 1:40) to isolate a white solid (1.37 g) (with a yield of 48%).

¹H NMR (400 MHz, MeOD-d4) δ 8.23 (d, *J =* 2.2 Hz, 1H), 8.05 (dd, *J =* 8.7, 2.3 Hz, 1H), 6.97 (d, *J =* 8.8 Hz, 1H), 4.14 - 4.06 (m, 2H), 3.87 (s, 3H), 1.85 - 1.74 (m, 2H), 1.64 - 1.51 (m, 2H), 1.44 - 1.32 (m, 16H), 1.28 - 1.20 (m, 3H).

### Preparation Example 9

This preparation example provides Compound 30, and the method for preparing Compound 30 includes the following steps.

### Step K coupling reaction:

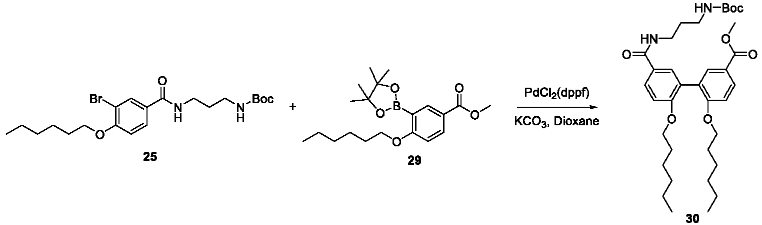

Tert-butyl (3-(3-bromo-4-hexyloxy)benzamide)propylaminocarbonate (0.9 g, 1.97 mmol), methyl 4-hexyloxy-3-(boronic acid pinacol ester)benzoate (0.86 g, 2.37 mmol), and potassium carbonate (0.82 g, 5.92 mmol) were added to 1,4-dioxane (30 mL), respectively. The reaction solution was degassed with argon, 1,1'-bisdiphenylphosphinoferrocene palladium dichloride (0.14 g, 0.2 mmol) was added, and the reaction solution was stirred at 90 °C for 16 h. Water (50 mL) was added to the reaction solution, and the reaction solution was extracted with ethyl acetate (50 mL × 3) and backwashed with saturated sodium chloride solution. The organic phases were dried with anhydrous sodium sulfate and concentrated. The resulting crude product was purified by column chromatography (ethyl acetate:dichloromethane = 1:40 → 1:5) to isolate a white solid **30** (1.15 g) (with a yield of 95%). [M+H]⁺: 613.4.

### Preparation Example 10

This preparation example provides Compound 31, and the method for preparing Compound 31 includes the following steps.

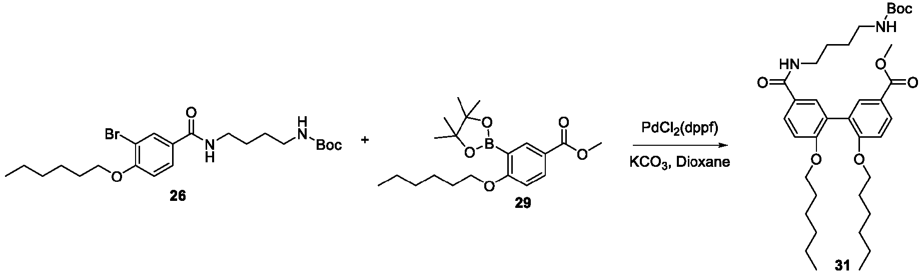

Tert-butyl (3-(3-bromo-4-hexyloxy)benzamide)butylaminocarbonate **26** (0.78 g, 1.66 mmol), methyl 4-hexyloxy-3-(boronic acid pinacol ester)benzoate (0.5 g, 1.38 mmol), and potassium carbonate (0.57 g, 4.14 mmol) were added to 1,4-dioxane (20 mL), respectively. The reaction solution was degassed with argon, 1,1'-bisdiphenylphosphinoferrocene palladium dichloride (0.1 g, 0.14 mmol) was added, and the reaction solution was stirred at 90 °C for 16 h. Water (50 mL) was added to the reaction solution, and the reaction solution was extracted with ethyl acetate (50 mL × 3) and backwashed with saturated sodium chloride solution. The organic phases were dried with anhydrous sodium sulfate and concentrated. The resulting crude product was purified by column chromatography (ethyl acetate:dichloromethane = 1:40 → 1:5) to isolate a white solid **31** (0.81g) (with a yield of 94%).

¹H NMR (400 MHz, MeOD-d4) δ 7.98 (dd, *J =* 8.7, 2.3 Hz, 1H), 7.82 (dt, *J* = 7.8, 1.7 Hz, 2H), 7.67 (d, *J =* 2.4 Hz, 1H), 7.04 (dd, *J =* 8.7, 4.5 Hz, 2H), 3.97 (dd, *J =* 11.1, 6.2 Hz, 4H), 3.84 (s, 3H), 3.34 (t, *J =* 6.8 Hz, 2H), 3.04 (t, *J =* 6.8 Hz, 2H), 1.63 - 1.52 (m, 4H), 1.39 (s, 6H), 1.29 - 1.11 (m, 10H), 0.80 (ddd, *J =* 9.4, 5.6, 2.3 Hz, 6H).

### Preparation Example 11

This preparation example provides Compound 32, and the method for preparing Compound 32 includes the following steps.

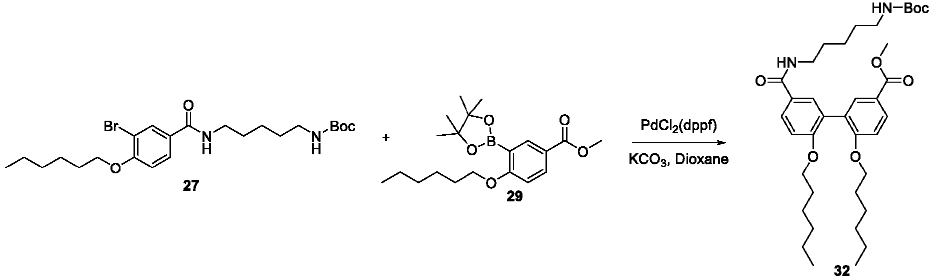

Tert-butyl (3-(3-bromo-4-hexyloxy)benzamide)pentylaminocarbonate **27** (1.22 g, 2.48 mmol), methyl 4-hexyloxy-3-(boronic acid pinacol ester)benzoate (1.08 g, 2.97mmol), and potassium carbonate (1.03 g, 7.44 mmol) were added to 1,4-dioxane (30 mL), respectively. The reaction solution was degassed with argon, 1,1'-bisdiphenylphosphinoferrocene palladium dichloride (0.18 g, 0.25 mmol) was added, and the reaction solution was stirred at 90 °C for 16 h. Water (50 mL) was added to the reaction solution, and the reaction solution was extracted with ethyl acetate (50 mL × 3) and backwashed with saturated sodium chloride solution. The organic phases were dried with anhydrous sodium sulfate and concentrated. The resulting crude product was purified by column chromatography (ethyl acetate:dichloromethane = 1:40 → 1:5) to isolate a yellow solid **32** (1.52 g) (with a yield of 95%).

### Preparation Example 12

This preparation example provides Compound 33, and the method for preparing Compound 33 includes the following steps.

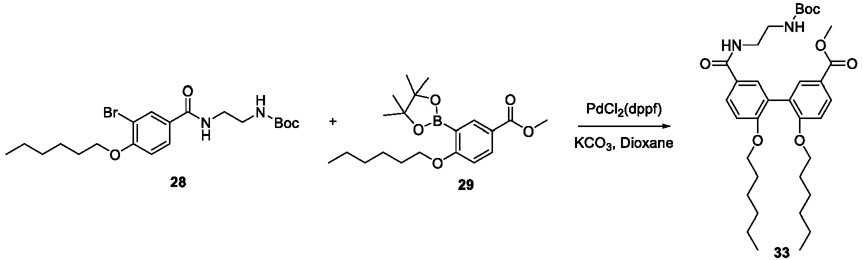

Tert-butyl (3-(3-bromo-4-hexyloxy)benzamide)-ethylaminocarbonate (1.07 g, 2.42 mmol), methyl 4-hexyloxy-3-(boronic acid pinacol ester)benzoate (1.05 g, 2.9 mmol), and potassium carbonate (1 g, 7.26 mmol) were added to 1,4-dioxane (30 mL), respectively. The reaction solution was degassed with argon, 1,1'-bisdiphenylphosphinoferrocene palladium dichloride (0.18 g, 0.25 mmol) was added, and the reaction solution was stirred at 90 °C for 16 h. Water (50 mL) was added to the reaction solution, and the reaction solution was extracted with ethyl acetate (50 mL × 3) and backwashed with saturated sodium chloride solution. The organic phases were dried with anhydrous sodium sulfate and concentrated. The resulting crude product was purified by column chromatography (ethyl acetate:dichloromethane = 1:40 → 1:5) to isolate a yellow solid **33** (1.18 g) (with a yield of 81%).

### Preparation Example 13

This preparation example provides Compound 34, and the method for preparing Compound 34 includes the following steps.

### Step K hydrolysis reaction:

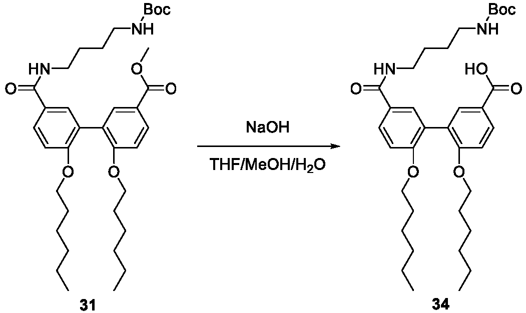

Methyl 5'-((3-((tert-butoxycarbonyl)amino)butyl)carbamoyl)-2',6-bis(hexyloxy)-[1,1'-biphenyl]-3-carboxylate 31 (0.9 g, 1.44 mmol) and sodium hydroxide (0.12 g, 2.88 mmol) were added to a mixed solution of tetrahydrofuran/methanol/water (8 mL/4 mL/6 mL), respectively, and stirred at 50 °C for 16 h. The reaction solution was concentrated under reduced pressure, water (30 mL) was added, and the pH of the reaction solution was adjusted to 5 with hydrochloric acid. The reaction solution was extracted with dichloromethane (20 mL × 3) and backwashed with saturated sodium chloride solution. The organic phases were dried with anhydrous sodium sulfate and concentrated to give a crude product **34** (0.26 g) as a yellow oil.

### Preparation Example 14

This preparation example provides Compound 35, and the method for preparing Compound 35 includes the following steps.

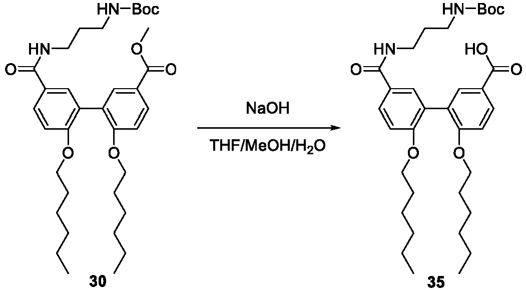

Methyl 5'-((3-((tert-butoxycarbonyl)amino)propyl)carbamoyl)-2',6-bis(hexyloxy)-[1,1'-biphenyl]-3-carboxylate 30 (0.5 g, 0.82 mmol) and sodium hydroxide (0.16 g, 4.09 mmol) were added to a mixed solution of tetrahydrofuran/methanol/water (8 mL/4 mL/6 mL), respectively, and stirred at 50 °C for 16 h. The reaction solution was concentrated under reduced pressure, water (30 mL) was added, and the pH of the reaction solution was adjusted to 5 with hydrochloric acid. The reaction solution was extracted with dichloromethane (20 mL × 3) and backwashed with saturated sodium chloride solution. The organic phases were dried with anhydrous sodium sulfate and concentrated to give a crude product **35** (0.65 g) as a yellow oil.

### Preparation Example 15

This preparation example provides Compound 36, and the method for preparing Compound 36 includes the following steps.

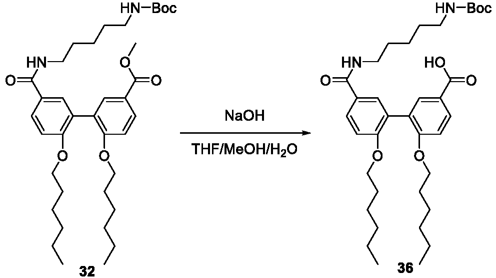

Methyl 5'-((3-((tert-butoxycarbonyl)amino)pentyl)carbamoyl)-2',6-bis(hexyloxy)-[1,1'-biphenyl]-3-carboxylate **32** (1.72 g, 2.69 mmol) and sodium hydroxide (0.22 g, 5.38 mmol) were added to a mixed solution of tetrahydrofuran/methanol/water (10 mL/8 mL/5 mL), respectively, and stirred at 50 °C for 16 h. The reaction solution was concentrated under reduced pressure, water (30 mL) was added, and the pH of the reaction solution was adjusted to 5 with hydrochloric acid. The reaction solution was extracted with dichloromethane (20 mL × 3) and backwashed with saturated sodium chloride solution. The organic phases were dried with anhydrous sodium sulfate and concentrated to give a crude product **36** (0.96 g) as a yellow solid.

### Preparation Example 16

This preparation example provides Compound 37, and the method for preparing Compound 37 includes the following steps.

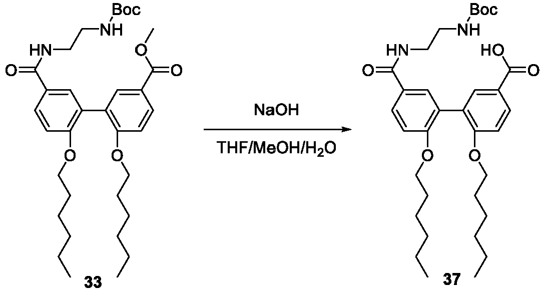

Methyl 5'-((3-((tert-butoxycarbonyl)amino)ethyl)carbamoyl)-2',6-bis(hexyloxy)-[1,1'-biphenyl]-3-carboxylate **33** (1.18 g, 1.97 mmol) and sodium hydroxide (0.16 g, 3.95 mmol) were added to a mixed solution of tetrahydrofuran/methanol/water (10 mL/8 mL/5 mL), respectively, and stirred at 50 °C for 16 h. The reaction solution was concentrated under reduced pressure, water (30 mL) was added, and the pH of the reaction solution was adjusted to 5 with hydrochloric acid. The reaction solution was extracted with dichloromethane (20 mL × 3) and backwashed with saturated sodium chloride solution. The organic phases were dried with anhydrous sodium sulfate and concentrated to give a crude product **37** (1.07 g) as a yellow solid.

### Preparation Example 17

This preparation example provides Compound 38, and the method for preparing Compound 38 includes the following steps.

### Step K hydrolysis of Boc:

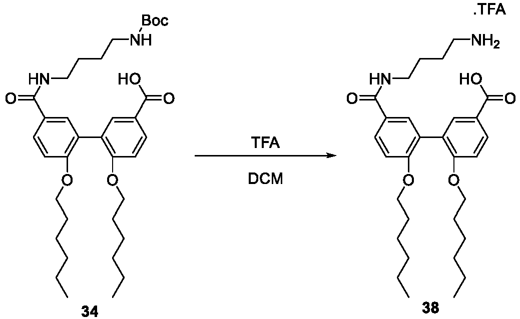

Trifluoroacetic acid (5 mL) was added to a dichloromethane solution (20 mL) of 5'-((3-((tert-butoxycarbonyl)amino)butyl)carbamoyl)-2',6-bis(hexyloxy)-[1,1'-biphenyl]-3-carboxylic acid 34 (0.26 g, 0.42 mmol). The reaction solution was stirred at room temperature for 6 h and concentrated under reduced pressure, and the resulting product **38** was directly used in the next reaction.

¹H NMR (500 MHz, MeOD-d4) δ 8.47 (brs, 1H), 7.99 (d, *J =* 8.0 Hz, 1H), 7.83 (d, *J =* 15.1 Hz, 2H), 7.68 (s, 1H), 7.03 (dd, *J =* 16.4, 8.5 Hz, 2H), 3.98 (s, 4H), 3.40 (s, 2H), 2.96 (s, 2H), 1.69-1.59 (m, 8H), 1.36 - 1.11 (m, 12H), 0.83 (s, 6H). [1/2M+H]⁺: 257.0.

### Preparation Example 18

This preparation example provides Compound 39, and the method for preparing Compound 39 includes the following steps.

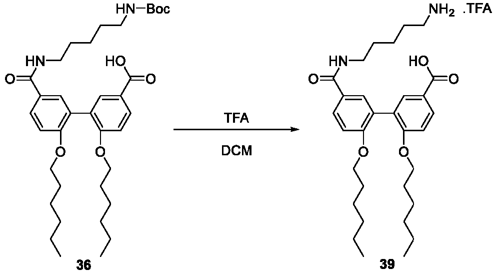

Trifluoroacetic acid (5 mL) was added to a dichloromethane solution (30 mL) of 5'-((3-((tert-butoxycarbonyl)amino)pentyl)carbamoyl)-2',6-bis(hexyloxy)-[1, 1'-biphenyl]-3-carboxylic acid 36 (0.96 g, 1.56 mmol). The reaction solution was stirred at room temperature for 6 h and concentrated under reduced pressure, and the resulting product **39** was directly used in the next reaction.

### Preparation Example 19

This preparation example provides Compound 40, and the method for preparing Compound 40 includes the following steps.

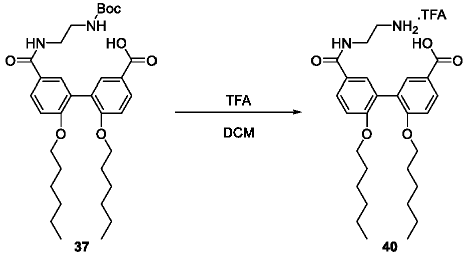

Trifluoroacetic acid (5 mL) was added to a dichloromethane solution (30 mL) of 5'-((3-((tert-butoxycarbonyl)amino)ethyl)carbamoyl)-2',6-bis(hexyloxy)-[1,1'-biphenyl]-3-carboxylic acid (1.07g, 1.83mmol). The reaction solution was stirred at room temperature for 6 h and concentrated under reduced pressure, and the resulting product 40 was directly used in the next reaction.

### Preparation Example 20

This preparation example provides Compound 41, and the method for preparing Compound 41 includes the following steps.

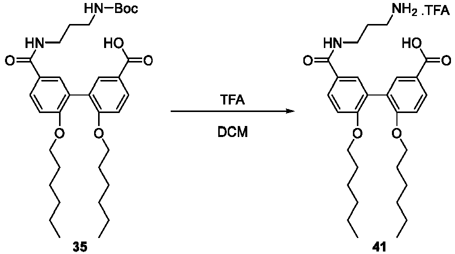

Trifluoroacetic acid (5 mL) was added to a dichloromethane solution (20 mL) of 5'-((3-((tert-butoxycarbonyl)amino)propyl)carbamoyl)-2',6-bis(hexyloxy)-[1,1'-biphenyl]-3-carboxylic acid 35 (650 mg, 1.1 mmol). The reaction solution was stirred at room temperature for 6 h and concentrated under reduced pressure, and the resulting product **41** was directly used in the next reaction.

### Example 1

This example provides Compound 1, and the method for preparing Compound 1 includes the following steps.

### Step C operation:

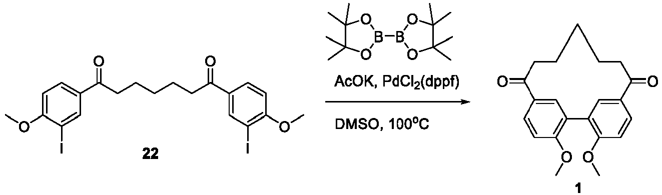

1,7-2(3-iodo-4-methoxyphenyl)heptane-1,7-dione 22 (5.92 g, 10 mmol), bis(pinacolato)diboron (3.06 g, 12 mmol), and potassium acetate (9.8 g, 100 mmol) were dissolved in dimethylsulfoxide (400 mL) at room temperature under argon protection, and [1,1'-bis(diphenylphosphino)ferrocene]palladium dichloride (0.732 g, 1 mmol) was added to the solution. The reaction solution was stirred at 100 °C for 7 h. 500 mL of water was added, and the resulting solid was filtered, washed with water, and purified by silica gel column chromatography (petroleum ether:ethyl acetate = 10:1 → 2:1) to isolate a white solid 1 (0.3 g) (with a yield of 8.9%).

¹H NMR (400 MHz, MeOD-d4) δ 8.04 (d, *J =* 2.5 Hz, 2H), 7.92 (dd, *J =* 8.7, 2.5 Hz, 2H), 7.19 (d, *J =* 8.7 Hz, 2H), 3.97 (s, 6H), 2.47 - 2.32 (m, 2H), 2.07 - 1.80 (m, 6H), 1.68 - 1.51 (m, 2H).

### Example 2

This example provides Compound 2, and the method for preparing Compound 2 includes the following steps.

### Step F operation:

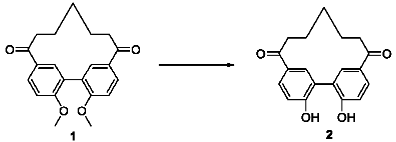

1⁶,2⁶-dimethoxy-1,2(1,3)-diphenylcyclononane-3,9-dione 1 (670 mg, 2 mmol) was dissolved in anhydrous dichloroethane (30 mL) at room temperature, and aluminum trichloride (801 mg, 6 mmol) was added to the solution. The reaction solution was stirred at 80 °C for 2 h. 30 mL of water was added. The resulting solid was filtered, collected, and washed with water. The resulting liquid was extracted with dichloromethane (20 mL × 3). The organic phases were combined, washed with saturated brine, dried with anhydrous sodium sulfate, and filtered. The resulting solution was concentrated under reduced pressure and purified and isolated through a silica gel preparation plate (dichloromethane:methanol = 15:1), and the resulting solids were combined and filtered to give an off-white solid 2 (370 mg) (with a yield of 60%).

¹H NMR (400 MHz, MeOD-d4) δ 8.21 (d, *J =* 2.4 Hz, 2H), 7.82 (dd, *J =* 8.5, 2.3 Hz, 2H), 7.01 (d, *J =* 8.5 Hz, 2H), 3.00 - 2.83 (m, 4H), 2.04-2.00 (m, 4H), 1.69 (m, 2H).

### Example 3

This example provides Compound 3, and the method for preparing Compound 3 includes the following steps.

### Step G operation:

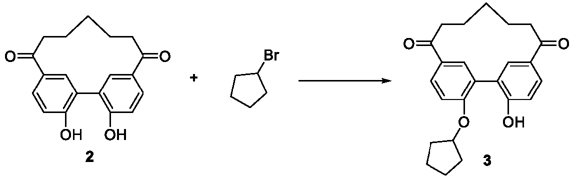

1⁶,2⁶-dihydroxy-1,2(1,3)-diphenylcyclononane-3,9-dione 2 (40 mg, 0.13 mmol) and potassium carbonate (107 mg, 0.78 mmol) were dissolved in acetonitrile (10 mL) at room temperature, and cyclopentylbromide (33 mg, 0.22 mmol) was added to the solution. The reaction solution was stirred at 90 °C for 12 h. The reaction solution was concentrated under reduced pressure and purified through a silica gel preparation plate (dichloromethane:methanol = 30:1) to isolate an off-white solid 3 (20 mg) (with a yield of 41%). ¹H NMR (400 MHz, DMSO-d6) δ 10.15 (s, 1H), 7.99 (dd, *J =* 19.7, 2.3 Hz, 2H), 7.79 (dd, *J =* 8.6, 2.4 Hz, 1H), 7.67 (dd, *J =* 8.5, 2.3 Hz, 1H), 7.18 (d, *J =* 8.8 Hz, 1H), 6.98 (d, *J =* 8.5 Hz, 1H), 5.03 (s, 1H), 3.17 (d, *J* = 5*.*1 Hz, 1H), 2.76 (d, *J =* 73.8 Hz, 2H), 2.02 - 1.68 (m, 10H), 1.65 - 1.43 (m, 4H).

### Example 4

This example provides Compound 4, and the method for preparing Compound 4 includes the following steps.

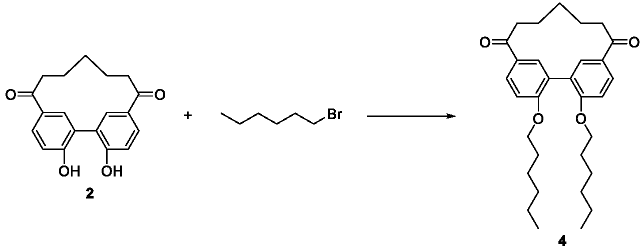

1⁶,2⁶-dihydroxy-1,2(1,3)-diphenylcyclononane-3,9-dione **2** (2.9 g, 9.35mmol) and potassium carbonate (7.75 g, 56.1 mmol) were dissolved in acetonitrile (100 mL) at room temperature, and bromohexane (9.26 g, 56.1 mmol) was added to the solution. The reaction solution was stirred at 90 °C for 12 h. The reaction solution was concentrated under reduced pressure and purified by silica gel column chromatography (dichloromethane:methanol = 100:1) to isolate an off-white solid **4** (1.79 g) (with a yield of 40%). ¹H NMR (400 MHz, MeOD-d4) δ 8.02 (d, *J =* 2.5 Hz, 2H), 7.89 (dd, *J =* 8.7, 2.4 Hz, 2H), 7.15 (d, *J =* 8.7 Hz, 2H), 4.23-4.20 (m, 2H), 4.13 - 4.02 (m, 2H), 3.42-3.34 (m, 2H), 2.38 (dd, *J =* 20.5, 10.2 Hz, 2H), 1.96-1.76 (m, 8H), 1.70 - 1.59 (m, 2H), 1.48-1.41 (m, 4H), 1.36 - 1.22 (m, 8H), 0.93-0.84 (m, 6H).

### Example 5

This example provides Compound 5, and the method for preparing Compound 5 includes the following steps.

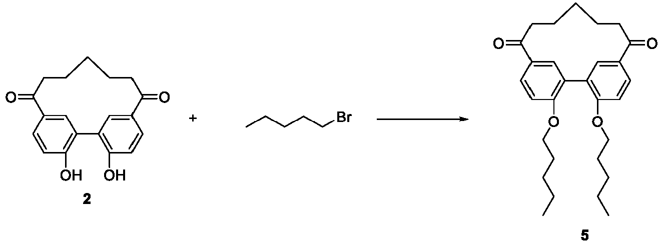

1⁶,2⁶-dihydroxy-1,2(1,3)-diphenylcyclononane-3,9-dione 2 (31 mg, 0.1 mmol) and potassium carbonate (83 mg, 0.6 mmol) were dissolved in acetonitrile (10 mL) at room temperature, and bromopentane (90 mg, 0.6 mmol) was added to the solution. The reaction solution was stirred at 90 °C for 12 h. The reaction solution was concentrated under reduced pressure and purified through a silica gel preparation plate (dichloromethane:methanol = 70:1) to isolate a white solid 5 (14 mg) (with a yield of 31%). ¹H NMR (400 MHz, MeOD-d4) δ 7.98 (d, *J =* 2.4 Hz, 2H), 7.87 (dd, *J =* 8.7, 2.4 Hz, 2H), 7.12 (d, *J =* 8.7 Hz, 2H), 4.18-4.03 (m, 4H), 3.39-3.33 (m, 2H), 2.36 (dd, *J =* 20.4, 10.3 Hz, 2H), 2.00 - 1.74 (m, 8H), 1.63-1.60 (m, 2H), 1.46 - 1.32 (m, 8H), 0.89 (t, *J =* 7.2 Hz, 6H).

### Example 6

This example provides Compound 6, and the method for preparing Compound 6 includes the following steps.

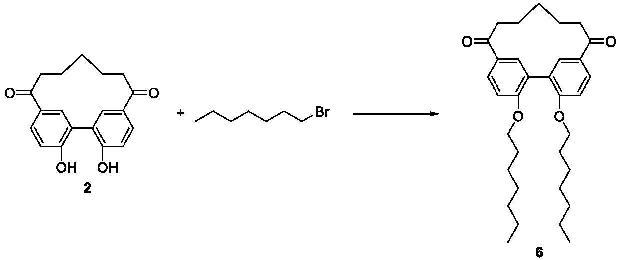

1⁶,2⁶-dihydroxy-1,2(1,3)-diphenylcyclononane-3,9-dione **2** (31 mg, 0.1 mmol) and potassium carbonate (110 mg, 0.8 mmol) were dissolved in acetonitrile (20 mL) at room temperature, and bromoheptane (144 mg, 0.8 mmol) was added to the solution. The reaction solution was stirred at 90 °C for 12 h. The reaction solution was concentrated under reduced pressure and purified through a silica gel preparation plate (dichloromethane:methanol = 70:1) to isolate a white solid **6** (16 mg) (with a yield of 32%).

¹H NMR (400 MHz, MeOD-d4) δ 7.99 (d, *J =* 2.4 Hz, 2H), 7.88 (dd, *J =* 8.7, 2.4 Hz, 2H), 7.13 (d, *J =* 8.7 Hz, 2H), 4.21-4.03 (m, 4H), 3.44 - 3.36 (m, 2H), 2.37 (dd, *J =* 20.5, 10.2 Hz, 2H), 2.04 - 1.74 (m, 8H), 1.69 - 1.56 (m, 2H), 1.46-1.40 (m, 4H), 1.38 - 1.24 (m, 12H), 0.86 (t, *J =* 6.9 Hz, 6H).

### Example 7

This example provides Compound 7, and the method for preparing Compound 7 includes the following steps.

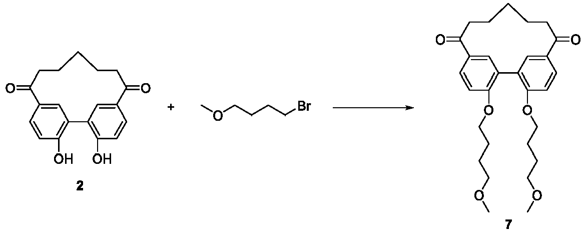

1⁶,2⁶-dihydroxy-1,2(1,3)-diphenylcyclononane-3,9-dione 2 (31 mg, 0.1 mmol) and potassium carbonate (110 mg, 0.8 mmol) were dissolved in acetonitrile (20 mL) at room temperature, and 1-bromo-4-methoxybutane (135 mg, 0.8 mmol) was added to the solution. The reaction solution was stirred at 90 °C for 12 h. The reaction solution was concentrated under reduced pressure and purified through a silica gel preparation plate (dichloromethane:methanol = 70:1) to isolate a white solid **7** (15 mg) (with a yield of 31%).

¹H NMR (400 MHz, MeOD-d4) δ 7.98 (d, *J =* 2.5 Hz, 2H), 7.87 (dd, *J =* 8.7, 2.4 Hz, 2H), 7.13 (d, *J =* 8.7 Hz, 2H), 4.30 - 4.03 (m, 4H), 3.41-3.30 (m, 6H), 3.26 (s, 6H), 2.42 - 2.29 (m, 2H), 1.99 - 1.77 (m, 8H), 1.75 - 1.56 (m, 6H).

### Example 8

This example provides Compound 8, and the method for preparing Compound 8 includes the following steps.

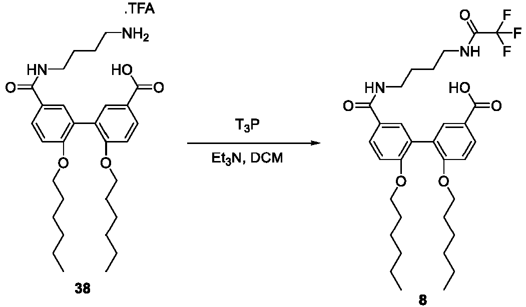

Triethylamine (1 mL) and propylphosphonic anhydride (50%) (800 mg) were added to a dichloromethane solution (10 mL) of 5'-((4-aminobutyl)carbamoyl)-2',6-bis(hexyloxy)-[1,1'-biphenyl]-3-carboxylic acid trifluoroacetate **38.** The reaction solution was stirred at room temperature for 3 days and concentrated under reduced pressure, and the resulting product was purified through a silica gel preparation plate (ethyl acetate:dichloromethane:acetic acid = 1:2:0.1) to give a white paste solid **8** (17 mg).

¹H NMR (400 MHz, MeOD-d4) δ 12.64 (s, 1H), 9.43 (s, 1H), 8.34 (s, 1H), 7.94-7.83 (m, 2H), 7.73 (dd, *J =* 9.1, 2.2 Hz, 2H), 7.20 - 6.98 (m, 2H), 3.99-3.94 (m, 4H), 3.24-3.19 (m, 4H), 1.52-1.51 (m, 8H), 1.23-1.16 (m, 12H), 0.81-0.77 (m, 6H).

### Example 9

This example provides Compound 9, and the method for preparing Compound 9 includes the following steps.

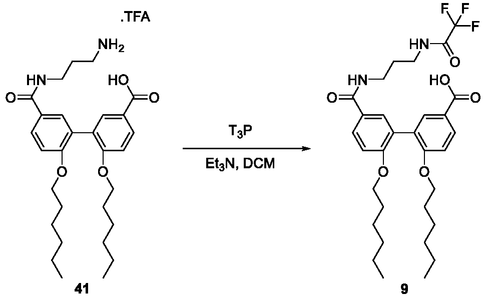

Triethylamine (1 mL) and propylphosphonic anhydride (50%) (2.2 g) were added to a dichloromethane solution (20 mL) of 5'-((4-aminopropyl)carbamoyl)-2',6-bis(hexyloxy)-[1,1'-biphenyl]-3-carboxylic acid trifluoroacetate **41.** The reaction solution was stirred at room temperature for 3 days and concentrated under reduced pressure, and the resulting product was purified through a silica gel preparation plate (ethyl acetate:dichloromethane:acetic acid = 1:2:0.1) to give a white paste solid **9** (30 mg).

¹H NMR (400 MHz, MeOD-d4) δ12.60 (s, 1H), 9.39 (s, 1H), 8.32 (s, 1H), 7.84 (ddd, *J* = 31.2, 8.6, 2.3 Hz, 2H), 7.69 (dd, *J =* 9.9, 9.0 Hz, 2H), 7.08 (dd, *J =* 9.9, 9.0 Hz, 2H), 3.93 (d, *J* = 6.3 Hz,4H), 3.20 (dd, *J =* 9.9, 6.3 Hz, 4H), 1.72-1.62 (m, 2H), 1.52 - 1.47 (m, 4H), 1.19-1.13 (m, 12H), 0.76-0.73 (dd, *J =* 7.0, 6.1 Hz, 6H).

### Example 10

This example provides Compound 10, and the method for preparing Compound 10 includes the following steps.

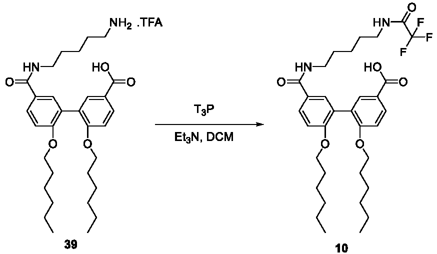

Triethylamine (5 mL) and propylphosphonic anhydride (50%) (3 g) were added to a dichloromethane solution (20 mL) of 5'-((4-aminopentyl)carbamoyl)-2',6-bis(hexyloxy)-[1,1'-biphenyl]-3-carboxylic acid trifluoroacetate **39.** The reaction solution was stirred at room temperature for 16 h and concentrated under reduced pressure, and the resulting product was purified through a silica gel preparation plate (ethyl acetate:dichloromethane:acetic acid = 1:2:0.1) to give a white solid **10** (20 mg).

¹H NMR (400 MHz, MeOD-d4) δ 8.34 (s, 1H), 8.01 (dd, *J =* 8.6, 2.2 Hz, 1H), 7.88 - 7.81 (m, 2H), 7.70 (d, *J =* 2.3 Hz, 1H), 7.06 (dd, *J =* 8.7, 1.4 Hz, 2H), 4.00 (td, *J =* 6.2, 3.6 Hz, 4H), 3.36 (dd, *J =* 9.5, 4.7 Hz, 2H), 3.27 (d, *J =* 7.2 Hz, 2H), 1.69 - 1.55 (m, 8H), 1.45 - 1.35 (m, 2H), 1.33 - 1.15 (m, 12H), 0.83 (t, *J =* 6.9 Hz, 6H).

### Example 11

This example provides Compound 11, and the method for preparing Compound 11 includes the following steps.

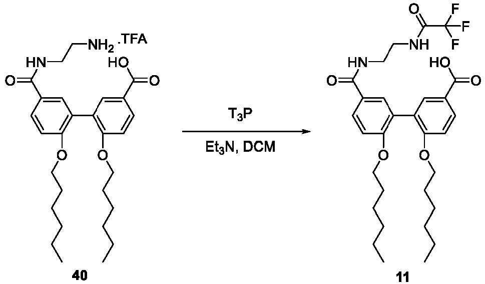

Triethylamine (5 mL) and propylphosphonic anhydride (50%) (3 g) were added to a dichloromethane solution (20 mL) of 5'-((4-aminoethyl)carbamoyl)-2',6-bis(hexyloxy)-[1,1'-biphenyl]-3-carboxylic acid trifluoroacetate **40.** The reaction solution was stirred at room temperature for 16 h and concentrated under reduced pressure, and the resulting product was purified through a silica gel preparation plate (ethyl acetate:dichloromethane:acetic acid = 1:2:0.1) to give a white solid **11** (18 mg).

¹H NMR (400 MHz, MeOD-d4) δ 8.01 (dd, *J =* 8.6, 2.1 Hz, 1H), 7.84 (dd, *J =* 8.9, 2.3 Hz, 2H), 7.70 (d, *J =* 2.4 Hz, 1H), 7.07 (dd, *J =* 8.7, 1.1 Hz, 2H), 4.00 (td, *J =* 6.2, 1.7 Hz, 4H), 3.51 (dd, *J =* 12.0, 4.8 Hz, 4H), 1.63-1.59 (m, 4H), 1.34 - 1.14 (m, 12H), 0.83 (td, *J =* 6.9, 1.4 Hz, 6H).

### Example 12

This example provides Compound 12, and the method for preparing Compound 12 includes the following steps.

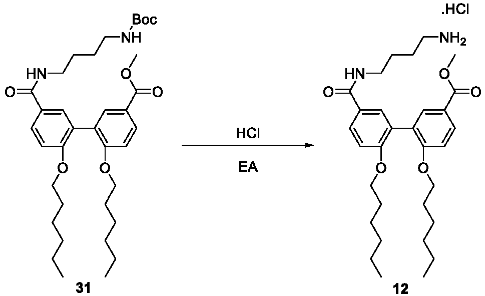

5'-((3-((tert-butoxycarbonyl)amino)butyl)carbamoyl)-2',6-bis(hexyloxy)-[1,1'-biphenyl]-3-carboxylate 31 (1.1 g, 1.76 mmol) was dissolved in a hydrochloric acid solution (80 mL) of ethyl acetate. The reaction solution was stirred at room temperature for 4 h and concentrated under reduced pressure to give a white solid **12** (900 mg) (with a yield of 91%).

¹H NMR (400 MHz, MeOD-d4) δ 8.48 (s, 1H), 8.06 (d, *J =* 8.1 Hz, 1H), 7.94 - 7.85 (m, 2H), 7.74 (brs, 1H), 7.12 (d, *J =* 7.7 Hz, 2H), 4.04 (d, *J =* 5.3 Hz, 4H), 3.91 (brs, 2H), 3.46 (brs, 2H), 1.75 (brs, 4H), 1.65 (brs, 4H), 1.28 (brs, 12H), 0.87 (d, *J =* 5.6 Hz, 6H).

### Example 13

This example provides Compound 13, and the method for preparing Compound 13 includes the following steps.

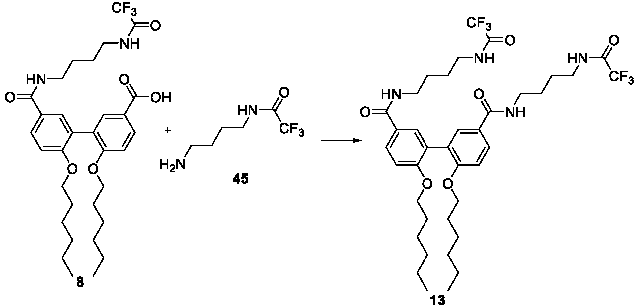

By referring to the method for preparing Compound 25 in Step I, Compound **8** (60 mg, 0.1 mmol) reacted with Compound **45** (20 mg, 0.11 mmol) to give Compound **13** as a white solid, 20 mg, with a yield of 26%.

¹H NMR (400 MHz, DMSO-*d*₆) δ 9.39 (s, 1H), 8.31 (s, 1H), 7.84 (d, *J =* 8.8 Hz, 1H), 7.69 (d, *J =* 15.3 Hz, 2H), 7.07 (d, *J =* 8.7 Hz, 1H), 4.21 (t, *J =* 6.5 Hz, 1H), 3.94 (t, *J =* 6.3 Hz, 2H), 3.30 (s, 2H), 3.21 (dd, *J =* 16.1, 6.5 Hz, 4H), 1.63 (t, *J =* 7.5 Hz, 1H), 1.50 (d, *J =* 16.7 Hz, 7H), 1.36 (q, *J =* 7.4 Hz, 2H), 1.20 (d, *J =* 22.6 Hz, 10H), 0.90 (t, *J =* 7.4 Hz, 2H), 0.83 - 0.72 (m, 4H).

### Example 14

This example provides Compound 14, and the method for preparing Compound 14 includes the following steps.

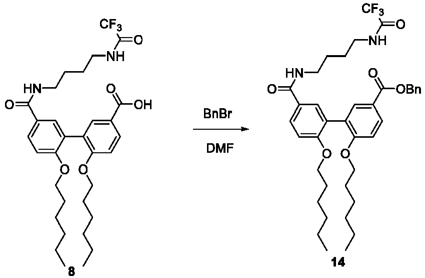

Cesium carbonate (63 mg, 0.2 mmol) was added to Compound **8** (60 mg, 0.1 mmol) in 5 mL of DMF solvent, benzyl bromide (0.1 mL) was added dropwise at room temperature, and the reaction was carried out at room temperature for 2 h. After the reaction was completed, water and ethyl acetate were added (20 mL) for liquid separation. The organic phases were collected, washed with saturated sodium chloride, dried with anhydrous sodium sulfate, filtered, concentrated, and purified by column chromatography to give Compound **14** as a light yellow solid, 60 mg, with a yield of 85.7%.

¹H NMR (400 MHz, Chloroform-*d*) δ 8.05 (dd, *J* = 8.6, 2.3 Hz, 1H), 7.93 (d, *J* = 2.2 Hz, 1H), 7.81 (dd, *J =* 8.6, 2.4 Hz, 1H), 7.55 (d, *J =* 2.3 Hz, 1H), 7.42 (d, *J* = 7.1 Hz, 2H), 7.39 - 7.28 (m, 3H), 7.09 (s, 1H), 6.92 (dd, *J =* 8.7, 5.5 Hz, 2H), 6.19 (s, 1H), 5.32 (s, 2H), 3.93 (q, *J =* 6.8 Hz, 4H), 3.44 (dd, *J =* 14.1, 6.3 Hz, 4H), 1.80 - 1.49 (m, 10H), 1.22 (d, *J =* 17.5 Hz, 14H), 0.81 (h, *J =* 3.7 Hz, 6H).

### Example 15

This example provides Compound 15, and the method for preparing Compound 15 includes the following steps.

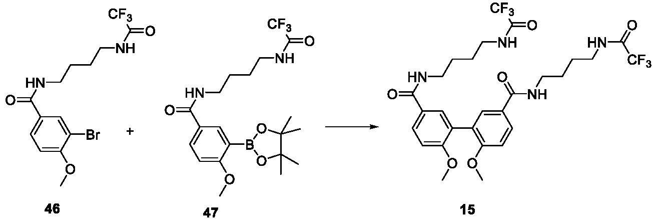

By referring to the coupling reaction in Step K and the method for preparing Compound 30, Compound **46** (40 mg, 0.1 mmol) reacted with Compound **47** (44 mg, 0.1 mmol) to give Compound **15** as a white solid, 20 mg, with a yield of 31.7%.

¹H NMR (400 MHz, Methanol-*d*₄) δ 7.95 (s, 1H), 7.84 (dd, *J =* 8.7, 2.4 Hz, 3H), 7.64 (d, *J =* 2.4 Hz, 2H), 7.49 (d, *J =* 1.3 Hz, 1H), 7.08 (d, *J =* 8.8 Hz, 3H), 3.77 (s, 9H), 3.35 (d, *J* = 9.5 Hz, 7H), 1.60 (p, *J =* 3.5 Hz, 15H).

ESI⁺, 634.2.

### Example 16

This example provides Compound 16, and the method for preparing Compound 16 includes the following steps.

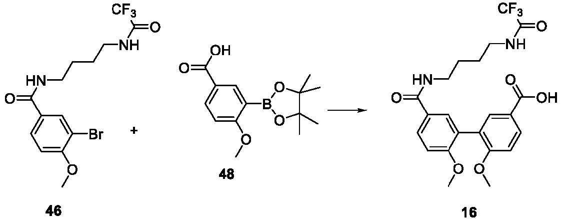

By referring to the coupling reaction in Step K and the method for preparing Compound 30, Compound **46** (40 mg, 0.1 mmol) reacted with Compound **48** (28 mg, 0.1 mmol) to give Compound 16 as a white solid, 25 mg, with a yield of 53.4%.

ESI⁺, 468.2.

### Example 17

This example provides Compound 17, and the method for preparing Compound 17 includes the following steps.

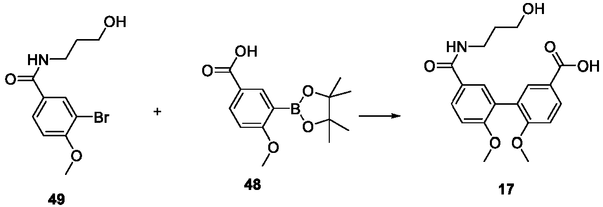

By referring to the coupling reaction in Step K and the method for preparing Compound 30, Compound **49** (30 mg, 0.1 mmol) reacted with Compound **48** (28 mg, 0.1 mmol) to give Compound 17 as a white solid, 26 mg, with a yield of 72.2%.

ESI⁺, 359.2.

### Example 18

This example provides Compound 18, and the method for preparing Compound 18 includes the following steps.

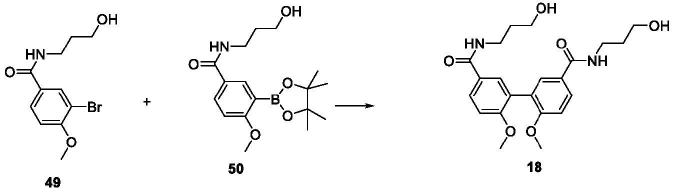

By referring to the coupling reaction in Step K and the method for preparing Compound 30, Compound **49** (30 mg, 0.1 mmol) reacted with Compound **48** (33 mg, 0.1 mmol) to give Compound **18** as a white solid, 16 mg, with a yield of 39%.

ESI⁺, 419.2.

### Example 19

This example provides Compound 19, and the method for preparing Compound 19 includes the following steps.

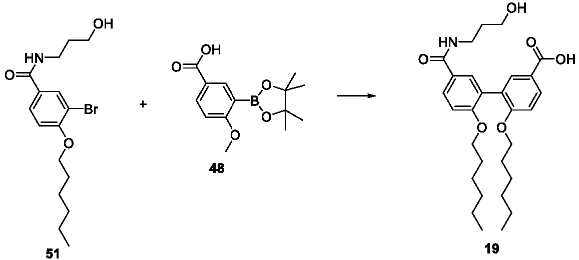

By referring to the coupling reaction in Step K and the method for preparing Compound **30,** Compound **51** (36 mg, 0.1 mmol) reacted with Compound **48** (33 mg, 0.1 mmol) to give Compound **19** as a white solid, 18 mg, with a yield of 36%.

HR-ESI (M+H) C₂₉H₄₂O₆N 500.3007, found 557.3030.

### Example 20

This example provides Compound 20, and the method for preparing Compound 20 includes the following steps.

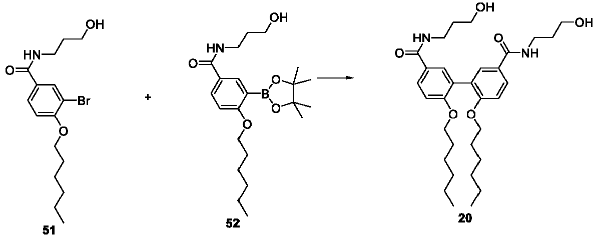

By referring to the coupling reaction in Step K and the method for preparing Compound 30, Compound 51 (36 mg, 0.1 mmol) reacted with Compound **48** (40 mg, 0.1 mmol) to give Compound 20 as a white solid, 20 mg, with a yield of 35.7%.

HR-ESI (M+H) C₃₂H₄₉O₆N₂ 557.3585, found 557.3605.

### Pharmacological experiment

### Experiment Example 1 Binding inhibition experiment of the biphenyl compound to LAG3 and its ligand MHCII

### (1) Experimental method

HTRF (Homogeneous time-resolved fluorescence) is based on two techniques, fluorescence resonance energy transfer (FRET) and time-resolved fluorescence (TRF). The detection was carried out using a 384-well plate. 2 µL of a compound to be tested with an appropriate concentration was collected, 4 µL of Tag1-LAG3 protein (with a final concentration of 10 nmol·L⁻¹) was added, 4 µL of Tag2-MHCII (with a final concentration of 20 nmol·L⁻¹) was added, and the plate was incubated at room temperature for 15 min. 10 µL of Anti-Tag1-Tb working solution (prepared by adding 25 µL of Anti-Tag1-Tab mother solution to 2475 µL of dilution buffer) was added, 10 µL of Anti-Tag2-XL665 working solution (prepared by adding 25 µL of Tag2-XL665 mother solution to 2475 µL of dilution buffer) was added, and the plate was sealed with a film and incubated at room temperature for 1 h. The detection was carried out using a Perkin Elma Envision instrument.

(2) The IC₅₀ of binding inhibition of the biphenyl compound to LAG3/MHCII was tested, and the test results are shown in Table 1 below.

**Table 1**

| Compound | µM | Inhibition rate (%) | IC₅₀ (µM) |
|---|---|---|---|
| 1 | 10 | 25.72 | - |
| 2 | 10 | 45.91 | - |
| 3 | - | - | 24.21 |
| 4 | - | - | 18.00 |
| 5 | - | - | 33.21 |
| 6 | - | - | 25.65 |
| 7 | - | - | 35.38 |
| 8 | - | - | 8.34 |
| 9 | - | - | 5.67 |
| 10 | - | - | 9.91 |
| 11 | - | - | 7.96 |
| 12 | - | - | > 100 |
| 13 | - | - | 4.01 |

(3) Summary: The IC₅₀ values of Compounds 8, 9, 10, 11, and 13 in Table 1 for inhibiting LAG3/MHCII interaction are all at the level of µmol·L⁻¹, indicating that these compounds have strong inhibitory effects on the binding of LAG3/MHCII.

### Experiment Example 2 Binding inhibition experiment of the biphenyl compound to LAG3 and its ligand FGL1

### (1) Experiment method

Reagent preparation: ① Tag1-LAG3 protein: a protein solution with a concentration of 50 nM (20 µL with a final system concentration of 10 nM) was prepared using a diluent. ② Tag2-FGL1 protein: a protein solution with a concentration of 100 nM (20 µL with a final system concentration of 20 nM) was prepared using a diluent. ③ Anti-Tag1-Tb: a 100× stock solution was diluted with a detection buffer to a 1 × stock solution. ④ Anti-Tag2-XL665: a 100× stock solution was diluted with a detection buffer to a 1 × stock solution.

Assay step: (1) 2 µL of compound solution (with a concentration to be measured of 10×) was added to a 384-well opaque plate having a white background, 4 µL of Tag1-LAG3 protein and 4 µL of Tag2-FGL1 protein were added, respectively, and the plate was incubated at room temperature for 15 min; (2) Anti-Tag1-Tb and Anti-Tag2-XL665 were mixed at a ratio of 1:1 in advance, and 10 µL of mix solution was added to each well; (3) the plate was gently mixed, sealed, and incubated at room temperature for 1 h or overnight before detection.

(2) The IC₅₀ values of binding inhibition of the biphenyl compound to LAG3/FGL1 are shown in Table 2 below.

**Table 2**

| Compound | IC₅₀ (µM) |
|---|---|
| 8 | 10.00 |
| 13 | 46.00 |
| 14 | - |

(3) Summary: The IC₅₀ value of Compound 8 in Table 2 for inhibiting LAG3/FGL1 interaction is 10 µmol·L⁻¹, indicating that Compound 8 has a strong inhibitory effect on the binding of LAG3/FGL1.

### Experiment Example 3 Experiment on the interaction of Compounds 4, 8, and 9 with LAG3 proteins of different species

### (1) Experiment method

### 1. Experiment principle

A surface plasmon is an electromagnetic wave on a metal surface and is generated by the interaction of photons and electrons which are freely vibrated. Surface plasmon resonance (SPR) is an optical phenomenon that occurs on the surfaces of two media, and such a phenomenon can be induced by photons or electrons. When light enters an optically thinner medium from an optically denser medium, the phenomenon of total reflection occurs, and an evanescent wave is generated and enters the optically thinner medium. When the totally reflected evanescent wave meets the plasmon wave on the metal surface, resonance may occur, the energy of the reflected light decreases, and a resonance peak occurs on the energy spectrum of the reflected light. Such a resonance is called surface plasmon resonance. The angle of incidence causing the surface plasmon resonance is called an SPR angle. An SPR biosensor provides a sensitive, non-labeled detection technique for monitoring intermolecular interactions in real time. The sensor detects the change in the SPR angle, and the SPR is related to the refractive index of the metal surface. When an analyte binds to the chip surface, the refractive index of the chip surface changes, thus causing the change in the SPR angle. This is the basic principle for the SPR biosensor to detect intermolecular interactions in real time. During the interaction analysis, the change in the SPR angle is recorded on the sensorgram in real time.

### 2. Method

Through the information query, the theoretical isoelectric point of the LAG3 protein is about 9.82, and the amino coupling method was contemplated to immobilize the protein. Since the main ligands of the LAG3 protein are FGL1 and MHCII proteins, after the LAG3 protein was coupled, the FGL1 protein sampling method was used to verify the protein activity.

Protein coupling buffer solution: 1.05× PBS-P+; 1.02× PBS-P+

Interaction buffer solution: 1.0× PBS-P+, 5% DMSO; 1.0× PBS-P+, 2% DMSO

### 3. Protein coupling

In this experiment, by using the CM5 chip amino coupling method, the human LAG3 protein was immobilized to the Fc2 or Fc3 channel, and the mouse LAG3 protein was immobilized to the Fc3 or Fc4 channel.

When interacting with Compound 8:

LAG3 protein coupling conditions were as follows: the concentration was about 25 µg/mL, the system was sodium acetate solution with a pH of 5.5, the chip activation time was 420 s, and the blocking time was 420 s.

When interacting with Compound 4 and Compound 9:

LAG3 protein coupling conditions were as follows: the concentration was about 50 µg/mL, the system was sodium acetate solution with a pH of 4.5, the chip activation time was 420 s, and the blocking time was 420 s.

### (2) Result

### 1. Interaction between the human LAG3 protein and Compound 4

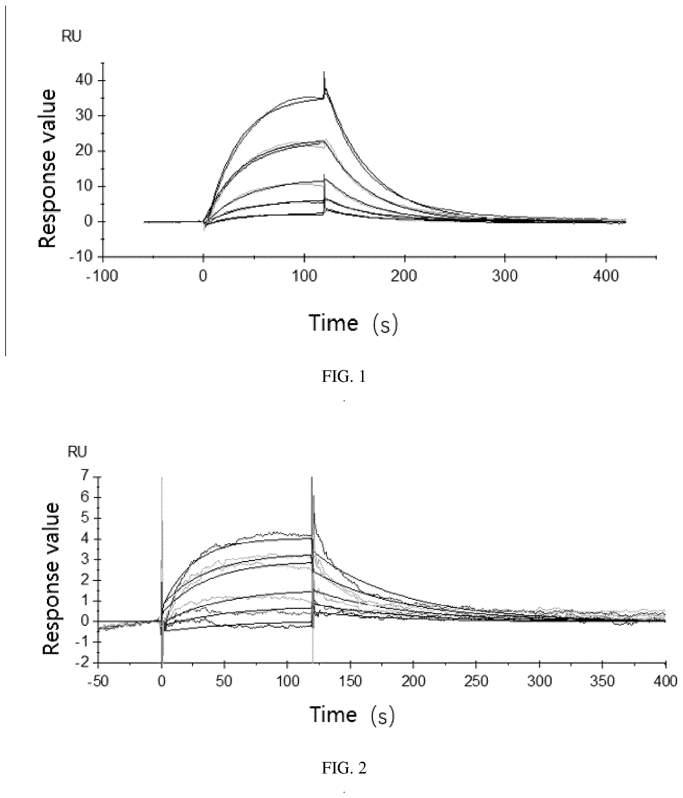

The coupling amount of the human LAG3 was approximately 5000 RU, and the theoretical Rmax was approximately 50 RU. The results of the assay indicate that Compound 4 interacts with the human LAG3 protein, and the kinetic constants are shown in FIG. 1.

### 2. Interaction between the mouse LAG3 protein and Compound 4

The coupling amount of the mouse LAG3 was approximately 5000 RU, and the theoretical Rmax was approximately 50 RU. The results of the assay indicate that Compound 4 interacts with the mouse LAG3 protein, and the kinetic constants are shown in FIG. 2.

### 3. Interaction between the human LAG3 protein and Compound 8

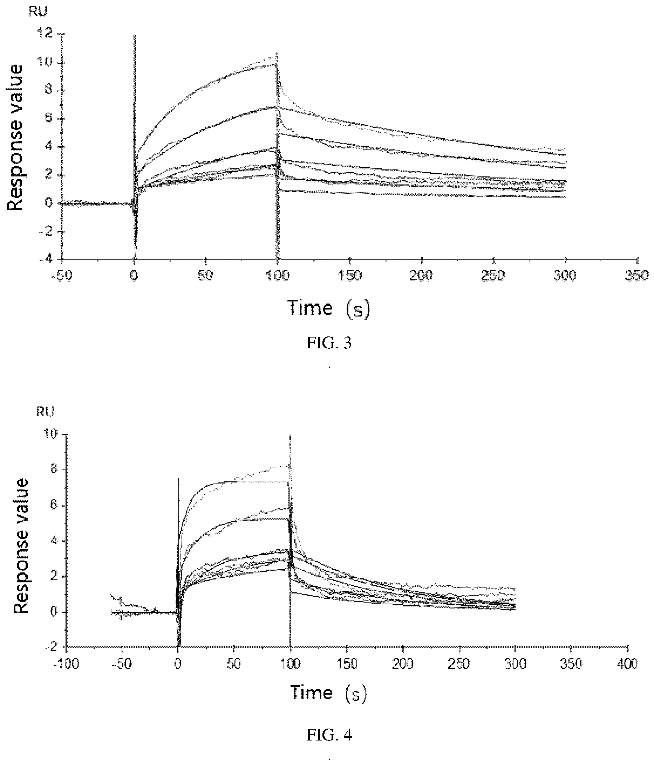

The coupling amount of the human LAG3 was approximately 3000 RU, and the theoretical Rmax was approximately 30 RU. The results of the assay indicate that Compound 8 interacts with the human LAG3 protein, and the kinetic constants are shown in FIG. 3.

### 4. Interaction between the mouse LAG3 protein and Compound 8

The coupling amount of the mouse LAG3 was approximately 3600 RU, and the theoretical Rmax was approximately 36 RU. The results of the assay indicate that Compound 8 interacts with the mouse LAG3 protein, and the kinetic constants are shown in FIG. 4.

### 5. Interaction between the human LAG3 protein and Compound 9

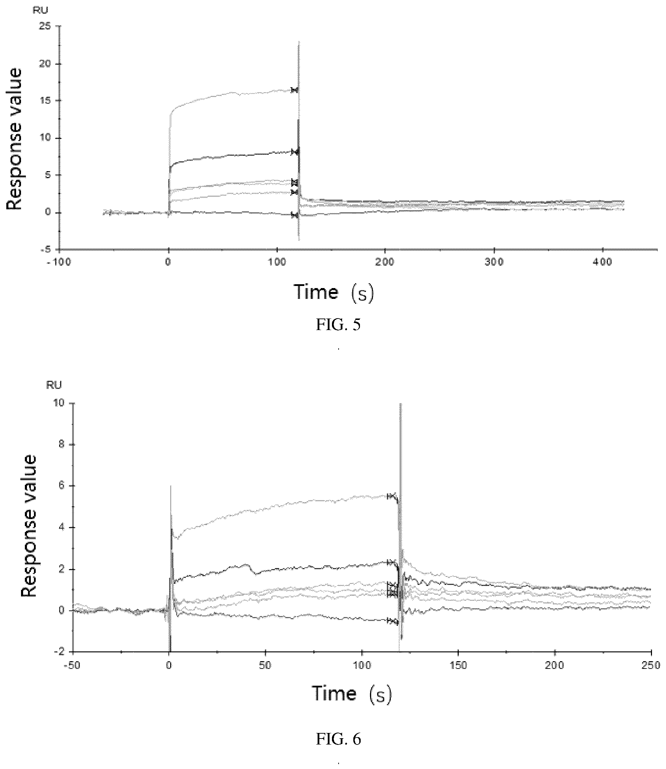

The coupling amount of the human LAG3 was approximately 5000 RU, and the theoretical Rmax was approximately 50 RU. The results of the assay indicate that Compound 9 interacts with the human LAG3 protein, and the kinetic constants are shown in FIG. 5. The interaction characteristics of "sudden rise and sudden drop" need to be fitted in a steady-state mode.

### 6. Interaction between the mouse LAG3 protein and Compound 9

The coupling amount of the mouse LAG3 was approximately 5000 RU, and the theoretical Rmax was approximately 50 RU. The results of the assay are shown in FIG. 6 and indicate that Compound 9 does not interact with the mouse LAG3 protein (KD = 0.68M).

### 7. Interaction between the human FGL1 protein and Compound 4

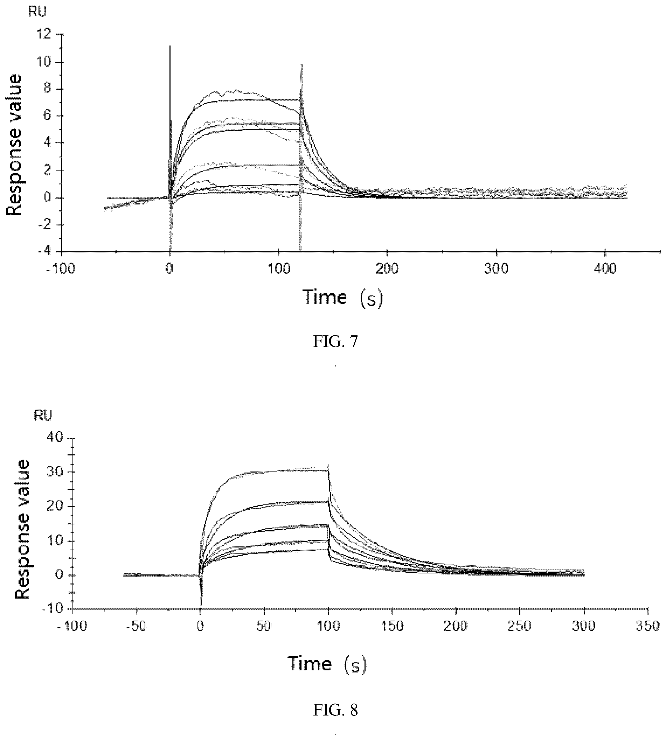

The coupling amount of the human FGL1 was approximately 5857 RU, and the theoretical Rmax was approximately 58 RU. The results of the assay show that Compound 4 interacts with the human FGL1 protein, and the kinetic constants are shown in FIG. 7.

### 8. Interaction between the human FGL1 protein and Compound 8

The coupling amount of the human FGL1 was approximately 5200 RU, and the theoretical Rmax was approximately 52 RU. The results of the assay indicate that Compound 8 interacts with the human FGL1 protein, and the kinetic constants are shown in FIG. 8.

### 9. Interaction between the human FGL1 protein and Compound 9

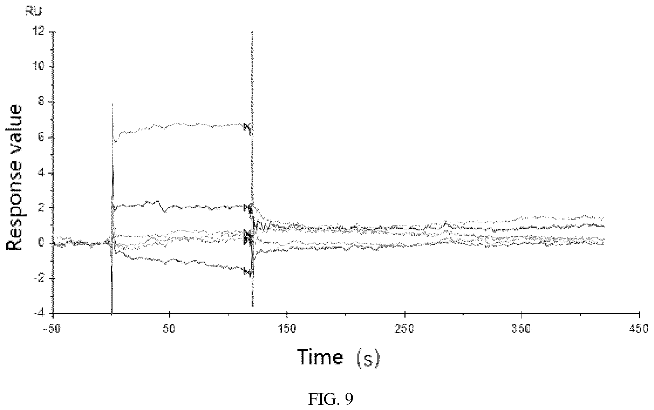

The coupling amount of the human FGL1 was approximately 5857 RU, and the theoretical Rmax was approximately 58 RU. The results of the assay indicate that Compound 9 does not interact with the human FGL1 protein, and the kinetic constants are shown in FIG. 9.

### (3) Summary

The purpose of this experiment is to determine the interaction affinity of LAG3 proteins of different species with Compounds 4, 8, and 9, and the determination is carried out by capturing the protein using a CM5 chip. The positive results of manual sampling of the FGL1 protein (5 µg/mL) indicate that the LAG3 protein activity under this interaction assay method is well maintained. The assay results of the binding affinity of the compound with the LAG3 protein are shown in Table 3, and the assay results of the binding affinity of the compound with the LAG3 protein are shown in Table 4.

**Table 3**

| Ligand | Analyte | Ka (1/Ms) | Kd (1/s) | KD (M) | Rmax (RU) | Note |
|---|---|---|---|---|---|---|
| Human LAG3 protein | Compound 4 | 417.7 | 2.209E-2 | 5.288E-5 | 124.7 | Bound |
| Mouse LAG3 protein | Compound 4 | 1061 | 1.399E-2 | 1.319E-5 | 5.214 | Bound |
| Human LAG3 protein | Compound 8 | 445.6 | 3.458E-3 | 7.761E-6 | 8.639 | Bound |
| Mouse LAG3 protein | Compound 8 | 1871 | 1.014E-2 | 5.419E-6 | 3.955 | Bound |
| Human LAG3 protein | Compound 9 | - | - | 3.240E-4 | 129.5 | Bound |
| Mouse LAG3 protein | Compound 9 | - | - | 0.6832 | 8.130 E+4 | Not bound |

**Table 4**

| Ligand | Analyte | Ka (1/Ms) | Kd (1/s) | KD (M) | Rmax (RU) | Note |
|---|---|---|---|---|---|---|
| FGL1 protein | Compound 4 | 2127 | 5.043E-2 | 2.322E-5 | 14.02 | Bound |
| FGL1 protein | Compound 8 | 1471 | 2.310E-2 | 1.570E-5 | 28.91 | Bound |
| FGL1 protein | Compound 9 | - | - | 2.059 | 3.346 E+5 | Not bound |

As can be seen from the data in Tables 3 and 4, the affinities of Compound 8 and Compound 4 for human- and murine-derived LAG3 proteins were essentially comparable (10⁻⁵ M to 10⁻⁶ M). The affinities of Compound 8 and Compound 4 with the new ligand FGL1 of LAG3 can also reach the level of 10⁻⁵ M.

### Experiment Example 4 Anti-tumor effects of Compounds 4 and 8 in vivo

### 1. Anti-tumor effect of Compound 4 on the mouse liver cancer HEPA1-6 in vivo

(1) 32 8-week-old female C57L mice were selected and divided into 5 cages. HEPA1-6 cells cultured with DMEM + 10% FBS *in vitro* were washed with PBS and digested with 0.25% trypsin for 3 min. The digestion was terminated with a serum-containing medium, the cells were centrifuged at 2500 rpm for 5 min, the supernatant was discarded, and the cells were resuspended with an appropriate amount of PBS twice. The number of cells was 1.14 × 10⁷ cells/mL as counted with a BioRad counting instrument. The cell suspension was placed in an Erlenmeyer flask in an ice bath for later use. The tumor fluid was evenly aspirated using a 1 mL sterile syringe and inoculated 0.2 mL/mouse. After the inoculation was completed, the mice continued to be fed under the initial feeding conditions.

(2) Animal grouping: On the second day after tumor fluid inoculation of C57B/L mice, 32 mice were randomly grouped: 8 mice in a control group, 6 mice in a Compound 4-5 mg/kg group, 6 mice in a Compound 4-10 mg/kg group, 6 mice in a Compound 4-20 mg/kg group, and 6 mice in a positive control drug cyclophosphamide (CTX)-100 mg/kg group. The drug test groups were given the drugs to be tested orally once a day, and the positive control drug CTX was injected intraperitoneally once. The mice were weighed and administered daily.

(3) Drug preparation: The corresponding amounts of the drugs to be tested were weighed according to the final concentrations of the experimental groups. The drugs of the Compound 4-5 mg/kg group, the Compound 4-10 mg/kg group, and the Compound 4-20 mg/kg group were prepared with 5% CMCNa + Tween 80, and CTX was prepared with normal saline. The mice in the control group were given 5% CMCNa solution orally. The dosages were as follows: 0.4 mL/20 g body weight of the animal for intragastric administration, and 0.2 mL/20 g body weight of the animal for intraperitoneal injection.

(4) The animals were sacrificed, and the tumors were separated and weighed. The animals in each group were treated immediately 1 h after administration on the last day and weighed, and the tumor tissue was separated and weighed.

### (5) Result

After 27 days of administration, the animals were sacrificed, and the tumors were separated and weighed. The growth inhibitory effect of Compound 4 on the mouse liver cancer HEPA1-6 *in vivo* is shown in Table 5.

**Table 5**

| Group | Dosage (mg/kg) | Tumor weight (g) | Inhibition rate (%) | P value |
|---|---|---|---|---|
| Blank control group | - | 0.678 ± 0.314 | NA | NA |
| CTX (ip) | 100 | 0.804 ± 0.459 | -18.65 | > 0.05 |
| Compound 4 (po) | 5 | 0.510 ± 0.312 | 24.72 | > 0.05 |
| Compound 4 (po) | 10 | 0.325 ± 0.256** | 52.00 | < 0.05 |
| Compound 4 (po) | 20 | 0.430 ± 0.140 | 36.56 | > 0.05 |

| | | | | |
|---|---|---|---|---|
| Note: ***P < 0.05, relative to the blank control group. | | | | |

As shown in Table 5, the tumor weight inhibition rates at the dosages of 5 mg/kg of Compound 4 and 10 mg/kg of Compound 4 were 24.72% and 52%, respectively, showing a certain dose dependence.

### 2. Anti-tumor effect of Compound 4 on the mouse melanoma B16F10 in vivo

(1) 43 8-week-old female C57B/L mice were selected and divided into 5 cages. One preserved B16F10 tumor-bearing female C57L mouse was sacrificed by cervical dislocation. 75% alcohol was sprayed on the skin of the mouse in a laminar flow cabinet, and the tumor tissue of the tumor-bearing mouse was separated by sterilized surgical instruments and cut into a sterilized 5 mL homogenizer with ophthalmic scissors. The tumor tissue was slowly milled on ice to form a tumor fluid. The number of cells was 6.85 × 10⁷ cells/mL as counted with a BioRad counting instrument. The tumor fluid was placed in an Erlenmeyer flask in an ice bath for later use. The tumor fluid was evenly aspirated using a 1 mL sterile syringe and inoculated 0.2 mL/C57/L mouse. After the inoculation was completed, the mice continued to be fed under the initial feeding conditions.

(2) Animal grouping and administration: On the second day after tumor fluid inoculation of C57B/L mice, 43 mice were randomly grouped: 10 mice in a control group, 8 mice in a Compound 4-20 mg/kg group, 8 mice in a Compound 4-30 mg/kg group, 8 mice in a Compound 4-40 mg/kg group, and 9 mice in a positive control drug cyclophosphamide CTX-100 mg/kg group. The mice were weighed and administered.

(3) Drug preparation: The corresponding amounts of the drugs were weighed according to the final concentrations of the experimental groups. The drugs of the Compound 4-20 mg/kg group and the Compound 4-30 mg/kg group were prepared with 0.25% CMCNa + Tween 80 and administered orally once a day. The drug of Compound 4-40 mg/kg was prepared with normal saline + Tween 80 and administered intraperitoneally. CTX was prepared with normal saline and administered intraperitoneally once. The mice in the control group were given 0.25% CMCNa solution orally.

(4) The animals were sacrificed, and the tumors were separated and weighed. The animals in each group were treated immediately 1 h after administration on the last day and weighed, and the tumor tissue was weighed.

### (5) Result

After 15 days of administration, the mice were sacrificed, and the tumors were separated and weighed. The growth inhibitory effect of Compound 4 on the mouse melanoma B16F10 *in vivo* is shown in Table 6.

**Table 6**

| Group | Dosage (mg/kg) | Tumor weight (g) | Inhibition rate (%) | P value |
|---|---|---|---|---|
| Blank control group | - | 2.906 ± 0.940 | NA | NA |
| CTX (ip) | 100 | 1.128 ± 0.394*** | 61.20 | < 0.01 |
| Compound 4 (po) | 20 | 1.304 ± 0.623*** | 55.12 | < 0.01 |
| Compound 4 (po) | 30 | 2.091 ± 1.199 | 28.00 | > 0.05 |
| Compound 4 (po) | 40 | 1.615 ± 0.495*** | 44.40 | < 0.01 |

| | | | | |
|---|---|---|---|---|
| Note: ***P < 0.01, relative to the blank control group. | | | | |

As shown in Table 6, the tumor weights in the group given Compound 4 at 20 mg/kg orally and the group given intraperitoneally injected with Compound 4 at 40 mg/kg were significantly smaller than the tumor weights in the blank control group (P < 0.01), indicating that the growth of mouse melanoma B16F10 can be inhibited at the above dosages.

### 3. Anti-tumor effect of Compound 8 on the mouse melanoma B16F10 in vivo

The anti-tumor effect of Compound 8 on the mouse melanoma B16F10 *in vivo* was observed in the same manner as described above.

Animal grouping and administration: On the second day after tumor fluid inoculation of C57B/L mice, tumor-bearing mice were randomly grouped: a control group, a Compound 8-5 mg/kg group, a Compound 8-10 mg/kg group, a Compound 8-20 mg/kg group, and a positive control drug cyclophosphamide CTX-100 mg/kg group. The animals were weighed and administered daily. The drugs to be tested were administered orally daily, and the positive control drug CTX was administered intraperitoneally once. After the experiment was completed, the body weights and tumor weights of the animals were weighed, and the tumor weight inhibition rate (%) was calculated.

The results of the growth inhibitory effect of Compound 8 on the mouse melanoma B16F10 *in vivo* are shown in Table 7.

**Table 7**

| Group | Dosage (mg/kg) | Tumor weight (g) | Inhibition rate (%) | P value |
|---|---|---|---|---|
| Blank control group | - | 1.80 ± 0.59 | NA | NA |
| CTX | 100 | 0.30 ± 0.10*** | 82.93 | < 0.01 |
| Compound 8 | 5 | 1.50 ± 0.27 | 16.94 | > 0.05 |
| Compound 8 | 10 | 1.45 ± 0.70 | 19.78 | > 0.05 |
| Compound 8 | 20 | 0.80 ± 0.46** | 53.64 | < 0.05 |

| | | | | |
|---|---|---|---|---|
| Note: **P < 0.05, and ***P < 0.01, relative to the blank control group. | | | | |

The results of the growth inhibitory effect of Compound 8 on the mouse melanoma B16F10 *in vivo* are shown in Table 7.As can be seen from the data in Table 7, Compound 8 has a significant inhibitory effect on the growth of mouse melanoma B16F10 when administered orally at 20 mg/kg, indicating that Compound 8 has a good anti-tumor effect.

The applicant has stated that although the processes and methods of the present application are described through the examples described above, the present application is not limited to the processes and steps described above, which means that the implementation of the present application does not necessarily depend on the processes and steps described above. It should be apparent to those skilled in the art that any improvements made to the present application, equivalent replacements of raw materials selected in the present application and additions of adjuvant ingredients thereof, and selections of specific methods, etc., all fall within the protection scope and the disclosed scope of the present application.

## Claims

1. A biphenyl compound, having a structure represented by Formula I: 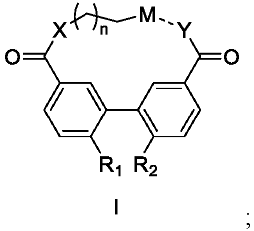
wherein R₁ and R₂ are each independently selected from any one of hydrogen, halogen, hydroxy, dimethylamino, cyano, nitro, C1-C8 alkoxycarbonyl, C1-C8 alkylcarbonyloxy, methylamino, methanesulfonyl, dimethylaminesulfonyl, amino, carboxyl, C1-C8 alkyl, C1-C8 alkoxy, C1-C8 trihaloalkyl, C1-C8 trihaloalkoxy, C1-C8 alkoxymethyleneoxy, C1-C8 methoxymethyleneoxy, cyclopentyloxy or cyclohexyloxy;
n is selected from 0, 1, 2, 3 or 4;
X is selected from CH₂, O or NH;
Y is selected from any one of CH₂, O, OH, NH, NH₂, NH(CH₂)_{n'}NH₂, NH(CH₂)_{n'}NHCOCF₃, OBn, C1-C8 alkyl, C1-C8 alkoxy, trihalomethylamino, dimethylamino, methylamino or trihaloalkoxy; wherein n' is selected from 1, 2, 3, 4 or 5;
M is selected from any one of CH₂, O, OH, NH, NH₂, trihalomethylamino, trihalomethylacyl, dimethylamino, methylamino, carboxyl, trihaloalkyl or C1-C8 alkoxymethyleneoxy; and
M is joined to Y by a bond or M does not form a bond with Y.

2. The biphenyl compound according to claim 1, wherein R₁ and R₂ are each independently selected from any one of hydrogen, fluorine, chlorine, bromine, hydroxy, dimethylamino, cyano, nitro, C1-C6 alkoxycarbonyl, C1-C6 alkylcarbonyloxy, methylamino, methanesulfonyl, dimethylaminesulfonyl, amino, carboxyl, C1-C6 alkyl, C1-C7 alkoxy, C1-C6 trihaloalkyl, C1-C7 trihaloalkoxy, C1-C6 alkoxymethyleneoxy, C1-C6 methoxymethyleneoxy, cyclopentyloxy or cyclohexyloxy;
n is selected from 1, 2, 3 or 4;
X is selected from CH₂, O or NH;
Y is selected from any one of CH₂, O, OH, NH, NH₂, NH(CH₂)_{n'}NH₂, NH(CH₂)_{n'}NHCOCF₃, OBn, C1-C6 alkyl, C1-C6 alkoxy, trihalomethylamino, dimethylamino, methylamino or trihaloalkoxy; wherein n' is selected from 2, 3, 4 or 5;
M is selected from any one of CH₂, O, OH, NH, NH₂, trihalomethylamino, trihalomethylacyl, dimethylamino, methylamino, carboxyl, trihaloalkyl or C1-C6 alkoxymethyleneoxy; and
M is joined to Y by a bond or M does not form a bond with Y.

3. The biphenyl compound according to claim 1 or 2, wherein R₁ and R₂ are each independently selected from any one of hydrogen, fluorine, chlorine, bromine, hydroxy, dimethylamino, cyano, nitro, C1-C4 alkoxycarbonyl, C1-C4 alkylcarbonyloxy, methylamino, methanesulfonyl, dimethylaminesulfonyl, amino, carboxyl, C1-C4 alkyl, C1-C7 alkoxy, C1-C4 trihaloalkyl, C1-C7 trihaloalkoxy, C1-C4 alkoxymethyleneoxy, C1-C4 methoxymethyleneoxy, cyclopentyloxy or cyclohexyloxy;
n is selected from 1, 2, 3 or 4;
X is selected from CH₂, O or NH;
Y is selected from any one of CH₂, O, OH, NH, NH₂, NH(CH₂)_{n'}NH₂, NH(CH₂)_{n'}NHCOCF₃, OBn, C1-C4 alkyl, C1-C4 alkoxy, trihalomethylamino, dimethylamino, methylamino or trihaloalkoxy; wherein n' is selected from 2, 3, 4 or 5;
M is selected from any one of CH₂, O, OH, NH, NH₂, trihalomethylamino, trihalomethylacyl, dimethylamino, methylamino, carboxyl, trihaloalkyl or C1-C4 alkoxymethyleneoxy; and
M is joined to Y by a bond or M does not form a bond with Y.

4. The biphenyl compound according to any one of claims 1 to 3, wherein R₁ and R₂ are each independently selected from any one of hydrogen, fluorine, chlorine, bromine, hydroxy, dimethylamino, cyano, nitro, methoxycarbonyl, ethoxycarbonyl, methylcarbonyloxy, ethoxycarbonyl, methylamino, methanesulfonyl, dimethylaminesulfonyl, amino, carboxyl, methyl, ethyl, methoxy, ethoxy, propoxy, 1-butanyloxy, 1-pentyloxy, 1-hexanyloxy, 1-heptanyloxy, 2-pentyloxy, 2-hexanyloxy, trifluoromethyl, trifluoroethyl, trifluoromethanyloxy, methoxymethyleneoxy, methoxyethyloxy, methoxypropoxy, methoxybutyloxy, cyclopentyloxy or cyclohexyloxy;
n is selected from 1, 2, 3 or 4;
X is selected from CH₂, O or NH;
Y is selected from any one of CH₂, O, OH, NH, NH₂, NH(CH₂)_{n'}NH₂, NH(CH₂)_{n'}NHCOCF₃, OBn, methyl, ethyl, methoxy, ethoxy, trifluoromethylamino, trichloromethylamino, dimethylamino, methylamino or trifluoromethanyloxy; wherein n' is selected from 2, 3, 4 or 5;
M is selected from any one of CH₂, O, OH, NH, NH₂, trifluoromethylamino, trichloromethylamino, trifluoromethylacyl, trichloromethylacyl, dimethylamino, methylamino, carboxyl, trifluoromethyl or methoxymethyleneoxy; and
M is joined to Y by a bond or M does not form a bond with Y.

5. The biphenyl compound according to any one of claims 1 to 4, wherein the compound is selected from any one of the following structures represented by 1 to 20:

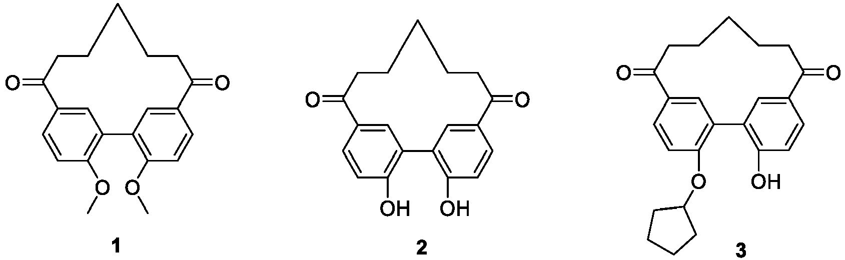

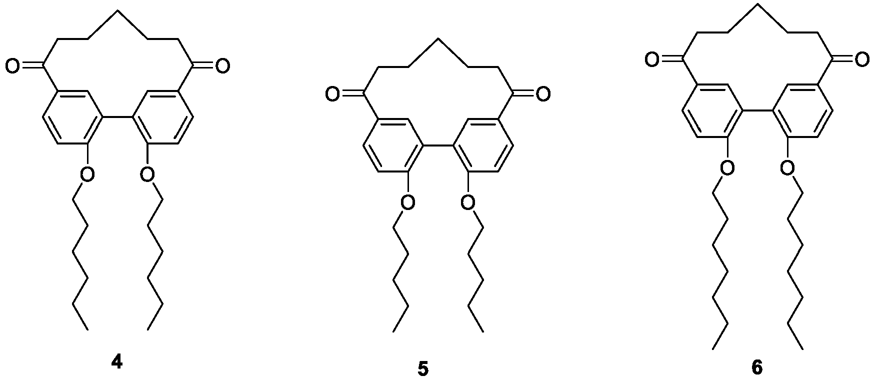

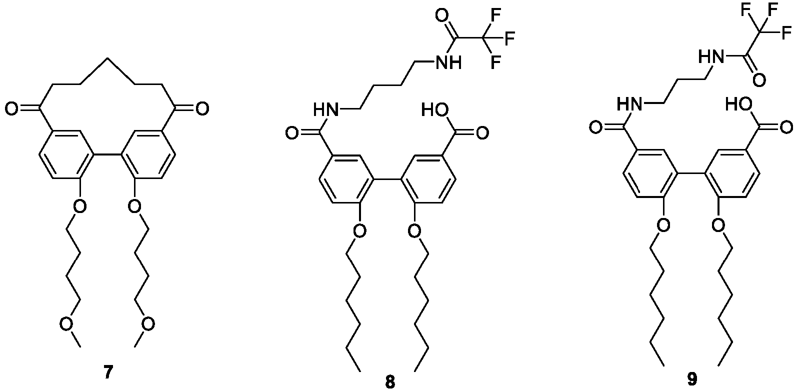

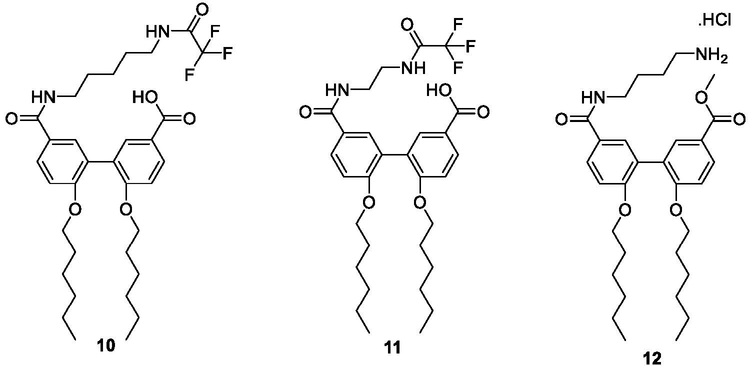

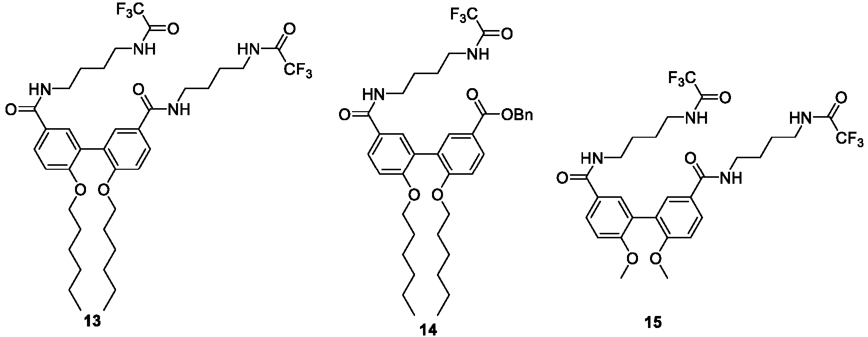

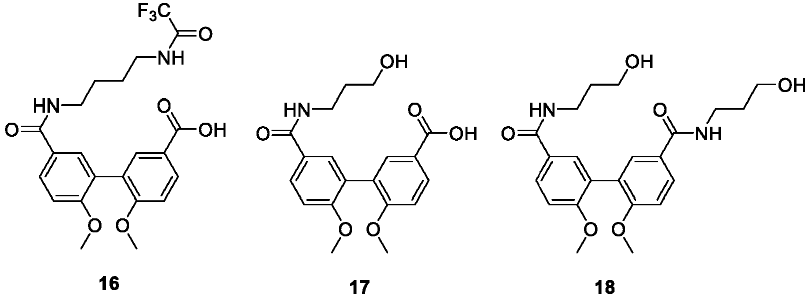

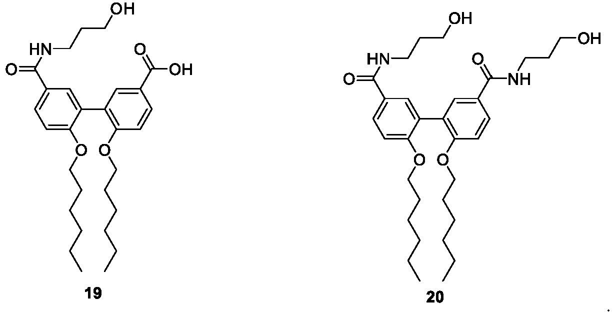

6. An isomer or a pharmaceutically acceptable salt of the biphenyl compound according to any one of claims 1 to 5;
preferably, the pharmaceutically acceptable salt comprises any one or a combination of at least two of a hydrochloride, a hydrobromide, a phosphate, a sulphate, a methanesulfonate, a p-toluenesulfonate, an acetate, a trifluoroacetate, a salicylate, an amino acid salt, a 2-O-β-D-Glucopyranosyl-L-ascorbic acid salt, a maleate, a tartrate, a fumarate, a citrate, a lactate, a sodium salt, a potassium salt, a calcium salt, a magnesium salt, a lithium salt, an ammonium salt, or a salt of an organic alkali capable of providing a physiologically acceptable cation;
preferably, the salt of an organic alkali capable of providing a physiologically acceptable cation comprises any one or a combination of at least two of a methylamine salt, a dimethylamine salt, a trimethylamine salt, a piperidine salt, a morpholine salt or a tris(2-hydroxyethyl)amine salt.

7. A method for preparing the biphenyl compound according to any one of claims 1 to 5, comprising the following steps:
synthesis method one:
(A) carrying out a nucleophilic addition reaction on a compound represented by Formula II, a compound represented by Formula III, and a compound represented by Formula IV to produce a compound represented by Formula V; wherein the reaction formula is as follows: 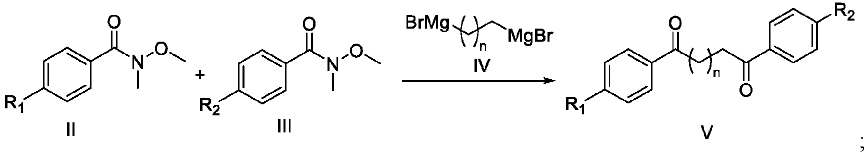
(B) carrying out an iodination reaction on the compound represented by Formula V and iodine to produce a compound represented by Formula VI; wherein the reaction formula is as follows: 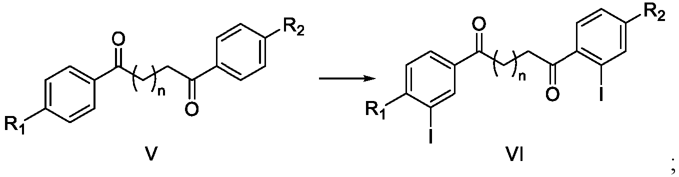
(C) carrying out a coupling reaction on the compound represented by Formula VI in an alkaline condition in the presence of zero-valent palladium to produce a compound represented by Formula I; wherein the reaction formula is as follows: 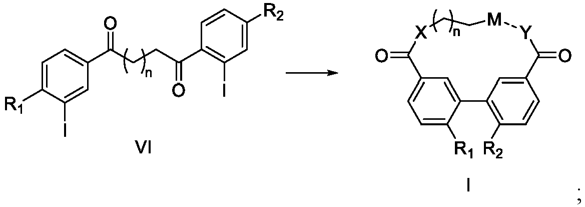 wherein R₁ and R₂ are each independently selected from any one of hydrogen, halogen, dimethylamino, cyano, nitro, C1-C8 alkoxycarbonyl, methylamino, methanesulfonyl, dimethylaminesulfonyl, amino, carboxyl, C1-C8 alkyl or C1-C8 trihaloalkyl; n is selected from 0, 1, 2, 3 or 4; X, Y, and M are each independently selected from CH₂;
or synthesis method two:
(D) carrying out a nucleophilic addition reaction on a compound represented by Formula II' and a compound represented by Formula III' to produce a compound represented by Formula IV', or carrying out a nucleophilic addition reaction on a compound represented by Formula II' and a compound represented by Formula II to produce a compound represented by Formula IV", or carrying out a nucleophilic addition reaction on a compound represented by Formula II' and a compound represented by Formula III to produce a compound represented by Formula IV‴; wherein the reaction formula is as follows: 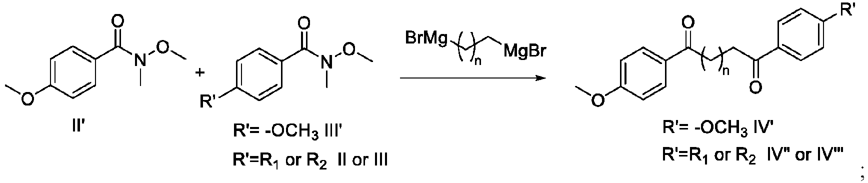
(E) carrying out an iodination reaction on the compound represented by Formula IV' to produce a compound represented by Formula V', or carrying out an iodination reaction on the compound represented by Formula IV" to produce a compound represented by Formula V", or carrying out an iodination reaction on the compound represented by Formula IV‴ to produce a compound represented by Formula V"'; wherein the reaction formula is as follows: 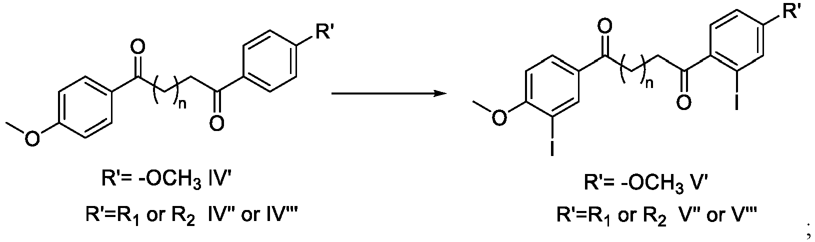
(F) carrying out a coupling reaction on the compound represented by Formula V' in an alkaline condition in the presence of zero-valent palladium, and carrying out hydrolysis in a Lewis acid condition to produce a compound represented by formula VI'; or carrying out a coupling reaction on the compound represented by Formula V" in an alkaline condition in the presence of zero-valent palladium, and carrying out hydrolysis in a Lewis acid condition to produce a compound represented by formula VI"; or carrying out a coupling reaction on the compound represented by Formula V‴ in an alkaline condition in the presence of zero-valent palladium, and carrying out hydrolysis in a Lewis acid condition to produce a compound represented by formula VI‴; wherein the reaction formula is as follows: 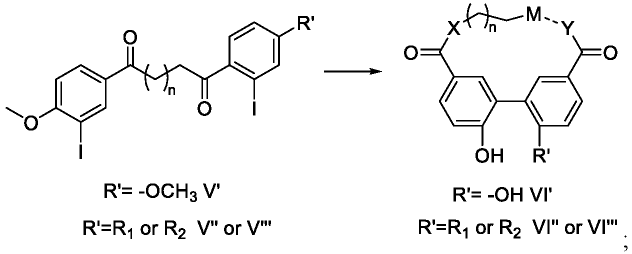
(G) carrying out an alkylation reaction on the compound represented by Formula VI' and an alkyl halide compound to produce a compound represented by Formula I; or carrying out an alkylation reaction on the compound represented by Formula VI" and an alkyl halide compound to produce a compound represented by Formula I; or carrying out an alkylation reaction on the compound represented by Formula VI‴ and an alkyl halide compound to produce a compound represented by Formula I; wherein the alkyl halide compound comprises an alkyl bromide or an alkyl chloride, and the reaction formula is as follows: 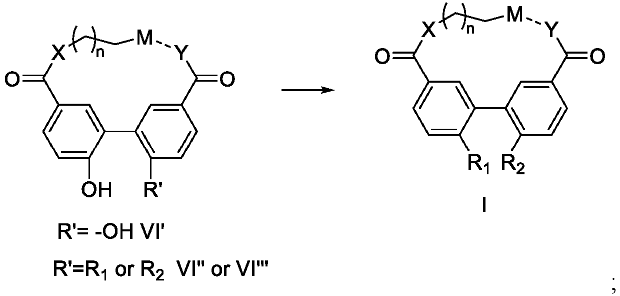 wherein R₁ and R₂ are each independently selected from any one of hydrogen, halogen, hydroxy, dimethylamino, cyano, nitro, C1-C8 alkoxycarbonyl, C1-C8 alkylcarbonyloxy, methylamino, methanesulfonyl, dimethylaminesulfonyl, amino, carboxyl, C1-C8 alkyl, C1-C8 alkoxy, C1-C8 trihaloalkyl, C1-C8 trihaloalkoxy, C1-C8 alkoxymethyleneoxy, C1-C8 methoxymethyleneoxy, cyclopentyloxy or cyclohexyloxy; n is selected from 0, 1, 2, 3 or 4; X, Y, and M are each independently selected from CH₂;
or synthesis method three:
(H) carrying out a hydrolysis reaction on a compound represented by Formula VII and alkali to produce a compound represented by Formula VIII; wherein the alkali comprises an organic alkali and/or an inorganic alkali, and the reaction formula is as follows: 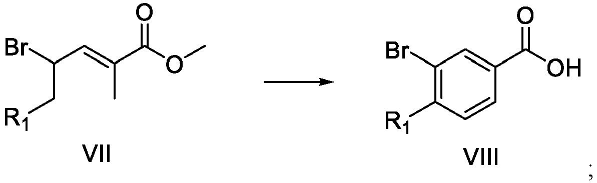
(I) carrying out a condensation reaction on the compound represented by Formula VIII and a compound represented by Formula IX in an alkaline condition to produce a compound represented by Formula X; wherein the reaction formula is as follows: 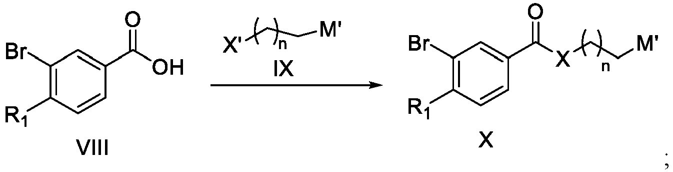
(J) carrying out a coupling reaction on the compound represented by Formula X to produce a compound represented by Formula XI; wherein the reaction formula is as follows: 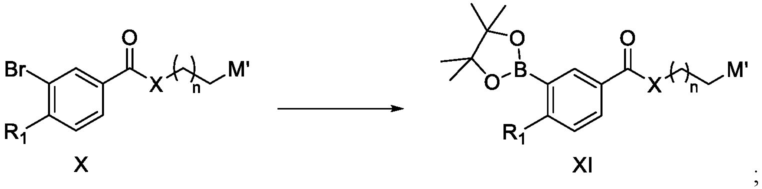
(K) carrying out a coupling reaction on the compound represented by Formula XI and a compound represented by Formula XII in an alkaline condition in the presence of zero-valent palladium to produce a compound represented by Formula I; or carrying out a coupling reaction on the compound represented by Formula XI and a compound represented by Formula XII in an alkaline condition in the presence of zero-valent palladium, carrying out hydrolysis in the presence of an organic alkali or an inorganic alkali after the reaction, stirring in a hydrochloric acid or a trifluoroacetic acid to obtain an amino hydrochloride salt, separating, and reacting with an acyl reagent to obtain a compound represented by Formula I; wherein the reaction formula is as follows: 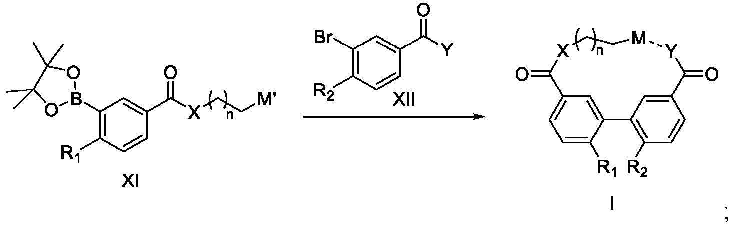
wherein R₁ and R₂ are each independently selected from any one of hydrogen, halogen, hydroxy, dimethylamino, cyano, nitro, C1-C8 alkoxycarbonyl, C1-C8 alkylcarbonyloxy, methylamino, methanesulfonyl, dimethylaminesulfonyl, amino, carboxyl, C1-C8 alkyl, C1-C8 alkoxy, C1-C8 trihaloalkyl, C1-C8 trihaloalkoxy, C1-C8 alkoxymethyleneoxy, C1-C8 methoxymethyleneoxy, cyclopentyloxy or cyclohexyloxy; n is selected from 0, 1, 2, 3 or 4; X is selected from CH₂, O or NH; X' is selected from OH or NH₂; Y is selected from any one of CH₂, O, OH, NH, NH₂, NH(CH₂)_{n'}NH₂, NH(CH₂)_{n'}NHCOCF₃,OBn, C₁₋₈ alkyl, C₁₋₈ alkoxy, trihalomethylamino, dimethylamino, methylamino or trihaloalkoxy, wherein n' is selected from 1, 2, 3, 4 or 5; and
M is selected from any one of CH₂, O, OH, NH, NH₂, trihalomethylamino, trihalomethylacyl, dimethylamino, methylamino, carboxyl, trihaloalkyl or C1-C8 alkoxymethyleneoxy, and M' is selected from any one of OH, NH₂, trihalomethylamino, trihalomethylacyl, dimethylamino, methylamino, carboxyl, trihaloalkyl or C1-C8 alkoxymethyleneoxy.

8. A pharmaceutical composition, comprising an active ingredient and a pharmacodynamically acceptable carrier, wherein the active ingredient comprises the biphenyl compound according to any one of claims 1 to 5 and/or the isomer or the pharmaceutically acceptable salt of the biphenyl compound according to claim 6;
preferably, a mass percentage of the active ingredient in the pharmaceutical composition is 0.1% to 95%.

9. Use of the biphenyl compound according to any one of claims 1 to 5, the isomer or the pharmaceutically acceptable salt of the biphenyl compound according to claim 6 or the pharmaceutical composition according to claim 8 in the preparation of a drug for preventing and/or treating a tumor, an autoimmune disease, an inflammatory disease, a neurodegenerative disease or a metabolic disease drug or an anti-aging drug.

10. The use according to claim 9, wherein the tumor is selected from any one or a combination of at least two of glioma, melanoma, gastric cancer, lung cancer, breast cancer, kidney cancer, liver cancer, oral epithelial carcinoma, head and neck tumor, cervical cancer, ovarian cancer, pancreatic cancer, prostate cancer, colon cancer, rectal adenocarcinoma, leukemia or lymphoma;
preferably, the autoimmune disease comprises any one or a combination of at least two of rheumatoid arthritis, systemic lupus erythematosus, ulcerative colitis, psoriasis, dermatitis or amyotrophic lateral sclerosis;
preferably, the inflammatory disease comprises any one or a combination of at least two of polyarteritis, phlebitis or reflux esophagitis;
preferably, the neurodegenerative disease comprises senile dementia and/or Parkinson's disease;
preferably, the metabolic disease comprises diabetes, hyperuricemia or gout.
